# EUROPEAN PATENT SPECIFICATION

(11) **EP 3 973 860 B1**
(45) Date of publication and mention of the grant of the patent: **08.07.2026**
(21) Application number: 20826836.7
(22) Date of filing: 17.06.2020
(51) Int. Cl.: A61B 5/318

(54) **ECG MONITORING METHOD AND WEARABLE DEVICE**
VERFAHREN ZUR EKG-ÜBERWACHUNG UND AM KÖRPER TRAGBARE VORRICHTUNG
PROCÉDÉ DE SURVEILLANCE ECG ET DISPOSITIF PORTÉ SUR SOI

(30) Priority: 18.06.2019 CN 201910525556
(43) Date of publication of application: 30.03.2022
(73) Proprietor: Huawei Technologies Co., Ltd., Longgang District Shenzhen, Guangdong 518129 (CN)
(72) Inventor: TAN, Xijin, Shenzhen, Guangdong 518129 (CN); XI, Yi, Shenzhen, Guangdong 518129 (CN); SUN, Shiyou, Shenzhen, Guangdong 518129 (CN); HU, Yi, Shenzhen, Guangdong 518129 (CN)
(74) Representative: Isarpatent
(86) International application number: PCT/CN2020/096590
(87) International publication number: WO 2020/253727

(56) References cited:
- CN-A- 105 249 951
- CN-A- 106 037 718
- CN-A- 106 073 707
- CN-A- 110 384 495
- US-A1- 2014 276 119
- US-A1- 2017 000 415
- US-A1- 2017 105 646
- US-A1- 2019 069 245
- US-B1- 10 154 460

## Description

### TECHNICAL FIELD

This disclosure relates to the field of terminal technologies, and the invention in particular relates to an ECG detection method and a wearable device.

### BACKGROUND

An electrocardiogram (electrocardiogram, ECG) may reflect a health status of a user. For example, the ECG may reflect a heart disease (such as arrhythmia). A conventional ECG technology is provided by only a hospital. If a user wants to perform ECG detection, the user needs to perform ECG detection at the hospital according to a specific procedure under instructions of a doctor. In addition, an existing medical system uses a fixed frequency when detecting an ECG. For example, a fixed frequency bandwidth is 0.05 Hz to 100 Hz. A medical device with the frequency bandwidth imposes a relatively strict requirement on a user (a user that receives ECG detection), for example, requires the user to lie still or have an empty stomach.

As a pace of life accelerates, a device that can detect an ECG anytime and anywhere to help a user monitor a health status of the user is increasingly needed by the user.

US 2014/276119 A1, US 2017/000415 A1, and US 2019/069245 A1 disclose wearable ECG measurement devices that can adjust their measurement settings based on data gathered from different sensors.

### SUMMARY

The object of the present application is to provide an ECG detection method and a wearable device. When a user wears the wearable device, regardless of a movement status such as a running state or a still state of the user, the wearable device can detect an ECG, for ease of use by the user. This object is solved by the attached independent claims and further embodiments and improvements of the invention are listed in the attached dependent claims. Hereinafter, up to the "brief description of the drawings", expressions like "...aspect according to the invention", "according to the invention", or "the present invention", relate to technical teaching of the broadest embodiment as claimed with the independent claims. Expressions like "implementation", "design", "optionally", "preferably", "scenario", "aspect" or similar relate to further embodiments as claimed, and expressions like "example", "...aspect according to an example", "the disclosure describes", or "the disclosure" describe technical teaching which relates to the understanding of the invention or its embodiments, which, however, is not claimed as such.

According to a first aspect an ECG detection method is provided. The method may be performed by a wearable device. The wearable device is, for example, a wristband or a watch. The wearable device may include a processor, a first electrode, and a second electrode. The method includes: the first electrode detects a first electrical signal, and the second electrode detects a second electrical signal; the processor determines a frequency bandwidth based on a current status of the wearable device and/or a status of a user in contact with the first electrode and the second electrode; and the processor determines an electrocardiogram ECG based on an electrical signal that is in the first electrical signal and the second electrical signal and whose frequency falls within the frequency bandwidth.

Usually, an electrical signal generated by a human body has a specific frequency, and a bandwidth of the frequency ranges from 0.05 Hz to 150 Hz. When the human body is in a moving state, respiration of the human body, an action of the body, and the like all affect generation of an electrical signal. Therefore, electrical signals (whose frequencies range from 0.05 Hz to 150 Hz) generated by the human body include a relatively large amount of noise. If an ECG detection device detects an ECG by using a fixed frequency domain bandwidth of frequencies 0.05 Hz to 150 Hz, accuracy of the detected ECG is low (because some of electrical signals within the frequency domain bandwidth of 0.05 Hz to 150 Hz are unavailable). Therefore, in this embodiment of this application, the wearable device may select an appropriate frequency bandwidth based on the status of the wearable device and/or the status of the user in contact with the first electrode and the second electrode, and then obtain the ECG based on the frequency bandwidth. Therefore, regardless of a movement status, such as a running state or a still state, of the user, the user can detect a relatively accurate ECG by using the wearable device, for ease of use by the user.

Further, the processor determines a frequency bandwidth based on a current status of the wearable device includes: the processor determines a current movement status of the wearable device based on a motion sensor in the wearable device; and the processor determines the frequency bandwidth based on the movement status.

It should be understood that the wearable device includes the motion sensor. The motion sensor is configured to detect the movement status of the wearable device. The wearable device selects an appropriate frequency bandwidth based on the movement status. Therefore, regardless of a movement status, such as a running state or a still state, of the user, the user detects a relatively accurate ECG by using the wearable device, for ease of use by the user.

In a possible design, that the processor determines a frequency bandwidth based on a status of a user in contact with the first electrode and the second electrode includes: the processor determines, based on an impedance value detected by a bioimpedance zinvasion Bio-z sensor in the wearable device, a skin status of a body portion of the user in contact with the first electrode and/or the second electrode; and the processor determines the frequency bandwidth based on the skin status of the user.

It should be understood that the wearable device may include the Bio-z sensor. The Bio-z sensor may be configured to detect the skin status of the body portion of the user in contact with the first electrode and/or the second electrode. The wearable device selects an appropriate frequency bandwidth based on the skin status. For example, when skin is quite dry or has quite a lot of water stains, a frequency bandwidth with a relatively small range may be selected; or when skin is moderately dry or moist, a frequency bandwidth with a relatively large range may be selected. In this method, regardless of a status (for example, a skin status) of the user, the user can detect a relatively accurate ECG by using the wearable device, for ease of operation by the user.

In a possible design, that the processor determines a frequency bandwidth based on a status of a user in contact with the first electrode and the second electrode includes: the processor determines a contact status of the user with the first electrode and the second electrode based on a pressure sensor and/or a capacitive sensor in the wearable device; and the processor determines the frequency bandwidth based on the contact status.

It should be understood that the wearable device may include the pressure sensor and/or the capacitive sensor. The pressure sensor and/or the capacitive sensor may be configured to detect the contact status of the user with the first electrode and the second electrode. The wearable device may select an appropriate frequency bandwidth based on the contact status. For example, in a case of relatively good contact, a frequency bandwidth with a relatively large range may be selected; or in a case of relatively poor contact, a frequency bandwidth with a relatively small range may be selected. In this method, regardless of a contact status of the user with the first electrode and the second electrode, the user can detect a relatively accurate ECG by using the wearable device, for ease of operation by the user.

In a possible design, before the first electrode detects the first electrical signal, and the second electrode detects the second electrical signal, a display of the wearable device displays an off-state lock screen, an on-state lock screen, or a home screen.

It should be understood that the wearable device may include the display. Before the user is in contact with the first electrode and the second electrode, the display may display the off-state lock screen, the on-state lock screen, or the home screen. That is, regardless of an interface displayed on the display of the wearable device, the first electrode and the second electrode can detect electrical signals provided that the user is in contact with the first electrode and the second electrode. The user does not need to be in contact with the first electrode and the second electrode after manually starting an ECG APP, for ease of operation by the user.

In a possible design, before the first electrode detects the first electrical signal, and the second electrode detects the second electrical signal, the wearable device may further detect an input operation; and enable an automatic ECG detection function in response to the input operation, where the first electrode and the second electrode are always in an enabled state after the wearable device enables the automatic ECG detection function.

In this embodiment of this application, the first electrode and the second electrode are always in the enabled state after the wearable device enables the automatic ECG detection function. It should be understood that, because the first electrode and the second electrode are always in the enabled state, regardless of an interface displayed on the display of the wearable device, the first electrode and the second electrode can detect electrical signals provided that the user is in contact with the first electrode and the second electrode. The user does not need to be in contact with the first electrode and the second electrode after manually starting an ECG APP, for ease of operation by the user.

In a possible design, the wearable device may further display a waveform corresponding to the ECG and health status information corresponding to the ECG.

It should be understood that the display of the wearable device may display the waveform corresponding to the ECG and the corresponding health status information, for ease of viewing by the user.

According to a second aspect a wearable device is provided. The wearable device includes a processor, a first electrode, and a second electrode. The first electrode is configured to detect a first electrical signal. The second electrode is configured to detect a second electrical signal. The processor is configured to determine a frequency bandwidth based on a current status of the wearable device and/or a status of a user in contact with the first electrode and the second electrode. The processor is further configured to determine an electrocardiogram ECG based on an electrical signal that is in the first electrical signal and the second electrical signal and whose frequency falls within the frequency bandwidth.

Further, the wearable device includes a motion sensor. The motion sensor is configured to detect a movement status of the wearable device. The processor is specifically configured to determine the frequency bandwidth based on the movement status.

In a possible design, the wearable device includes a bioimpedance zinvasion Bio-z sensor, and the Bio-z sensor is disposed on the first electrode and/or the second electrode. The Bio-z sensor is configured to detect a skin status of a body portion of the user in contact with the first electrode and/or the second electrode. The processor is specifically configured to determine the frequency bandwidth based on the skin status of the user.

In a possible design, the wearable device includes a pressure sensor and/or a capacitive sensor. The pressure sensor is disposed on the first electrode and/or the second electrode, and/or the capacitive sensor is disposed on the first electrode and/or the second electrode. The pressure sensor and/or the capacitive sensor are/is configured to detect a contact status of the user with the first electrode and/or the second electrode. The processor is specifically configured to determine the frequency bandwidth based on the contact status.

In a possible design, the wearable device further includes a display. Before the first electrode detects the first electrical signal, and the second electrode detects the second electrical signal, the display displays an off-state lock screen, an on-state lock screen, or a home screen.

In a possible design, the wearable device further includes an input device, and the input device is configured to detect an input operation. The processor is further configured to enable an automatic ECG detection function in response to the input operation, so that the first electrode and the second electrode are always in an enabled state.

In a possible design, the wearable device includes the display, and the display is configured to display a waveform corresponding to the ECG and health status information corresponding to the ECG.

According to a third not claimed aspect, a wearable device is provided, including a first electrode, a second electrode, at least one processor, and a memory. The memory is configured to store one or more computer programs. When the one or more computer programs stored in the memory are executed by the at least one processor, the electronic device is enabled to implement the method according to any one of the first aspect or the possible designs of the first aspect.

According to a fourth not claimed aspect, a wearable device is further provided. The wearable device includes modules/units that perform the method according to any one of the first aspect or the possible designs of the first aspect. These modules/units may be implemented by hardware, or may be implemented by hardware by executing corresponding software.

According to a fifth not claimed aspect, a computer-readable storage medium is further provided. The computer-readable storage medium includes a computer program. When the computer program runs on an electronic device, the electronic device is enabled to perform the method according to any one of the first aspect or the possible designs of the first aspect.

According to a sixth aspect a program product is further provided. When the program product runs on an electronic device, the electronic device is enabled to perform the method according to any one of the first aspect or the possible designs of the first aspect.

According to a seventh not claimed aspect, a chip is further provided. The chip is coupled to a memory in an electronic device, and is configured to invoke a computer program stored in the memory and perform the technical solution according to any one of the first aspect or the possible designs of the first aspect in the embodiments of this application. In this embodiment of this application, "coupled" means that two parts are directly or indirectly combined with each other.

Features/steps which in the preceding summary have been denoted as "according to the invention" or as "not claimed", are also to be understood in the same manner in the description below and in the attached drawings, even if this is not explicitly mentioned.

### BRIEF DESCRIPTION OF DRAWINGS

FIG. 1A is a schematic diagram of a hardware structure of a wearable device 100 according to an embodiment of this application;
FIG. 1B is a schematic diagram of a hardware structure of a wearable device 100 according to an embodiment of this application;
FIG. 2 is a schematic diagram of a structure of a wearable device 100 according to an embodiment of this application;
FIG. 3(a) to FIG. 3(c) each are a schematic diagram of a structure of a wearable device 100 according to an embodiment of this application;
FIG. 4 is a schematic diagram of a structure of a watch according to an embodiment of this application;
FIG. 5A is a schematic diagram of a structure of a watch according to an embodiment of this application;
FIG. 5B is a schematic diagram of a structure of a watch according to an embodiment of this application;
FIG. 6 is a schematic diagram of a structure of a watch according to an embodiment of this application;
FIG. 7 is a schematic diagram of an ECG waveform according to an embodiment of this application;
FIG. 8 is a schematic diagram in which a user wears a watch according to an embodiment of this application;
FIG. 9(a) to FIG. 9(c) each are a schematic diagram of a graphical user interface of a wearable device according to an embodiment of this application;
FIG. 10(a) and FIG. 10(b) each are a schematic diagram of a graphical user interface of a wearable device according to an embodiment of this application;
FIG. 11 is a schematic diagram of a graphical user interface of a wearable device according to an embodiment of this application;
FIG. 12(a) to FIG. 12(d) each are a schematic diagram of a graphical user interface of a mobile phone according to an embodiment of this application;
FIG. 13(a) to FIG. 13(d) each are a schematic diagram of a graphical user interface of a mobile phone according to an embodiment of this application; and
FIG. 14 is a schematic flowchart of an ECG detection method according to an embodiment of this application.

### DESCRIPTION OF EMBODIMENTS

The following describes the technical solutions in the embodiments of this application in detail with reference to the accompanying drawings in the following embodiments of this application.

The following first explains some terms in the embodiments of this application, for ease of understanding by a person skilled in the art.

It should be noted that a frequency bandwidth in the following embodiments of this application is used to indicate a frequency range for filtering an electrical signal of a human body. A filter may be disposed in a chip or a part (for example, a processor) that has an ECG detection function, or a chip or a part (for example, a processor) that has an ECG detection function may be connected to a filter. The filter may filter, by using a specific frequency bandwidth, an electrical signal that is input to the filter. Usually, an electrical signal generated by a human body has a specific frequency. It is determined, by using a test, that the frequency of the electrical signal of the human body falls within a range of 0.05 Hz to 150 Hz. Assuming that a filter filters electrical signals of a human body by using a frequency bandwidth of 7 Hz to 23 Hz, a frequency of an electrical signal retained after the filtering falls within the range of 7 Hz to 23 Hz, and an electrical signal outside the range is filtered out.

In the embodiments of this application, "at least one" means one or more, and "a plurality of" means two or more. In addition, it should be understood that, in the descriptions of this application, terms such as "first" and "second" are merely used for differentiation and description, but should not be understood as an indication or implication of relative importance or an indication or implication of an order.

Terms used in the following embodiments are merely intended to describe specific embodiments, but are not intended to limit this application. The terms "one", "a", "the", "the foregoing", "this", and "the one" of singular forms used in this specification and the appended claims of this application are also intended to include plural forms such as "one or more", unless otherwise specified in the context clearly. It should be further understood that, in the embodiments of this application, "one or more" means one, two, or more. In addition, "and/or" describes an association relationship between associated objects, and indicates that three relationships may exist. For example, A and/or B may indicate the following cases: Only A exists, both A and B exist, and only B exists, where A and B may be singular or plural. The character "/" usually indicates an "or" relationship between associated objects.

Reference to "one embodiment", "some embodiments", or the like described in this specification indicates that one or more embodiments of this application include a specific feature, structure, or characteristic described with reference to the embodiments. Therefore, in this specification, statements such as "in an embodiment", "in some embodiments", "in some other embodiments", and "in other embodiments" that appear at different places do not necessarily mean referencing a same embodiment, but mean "one or more but not all of the embodiments", unless otherwise specifically emphasized. The terms "include", "comprise", "have", and their variants all mean "include but are not limited to", unless otherwise specifically emphasized.

It should be noted that, usually, an electrical signal generated by a human body has a specific frequency, and a bandwidth of the frequency ranges from 0.05 Hz to 150 Hz. Electrical signals within a frequency domain bandwidth of 0.05 Hz to 150 Hz that are generated when the human body is in a still state include a relatively small amount of noise. Therefore, when the human body is in the still state, an electronic device may collect an ECG by using the frequency domain bandwidth of 0.05 Hz to 150 Hz because electrical signals generated by the human body include a relatively small amount of noise in the frequency domain bandwidth, and all electrical signals within the range of 0.05 Hz to 150 Hz are available. When the human body is in a moving state, respiration of the human body, an action of the body, and the like all affect generation of an electrical signal. Therefore, electrical signals within the range of 0.05 Hz to 150 Hz that are generated by the human body include a relatively large amount of noise. In this case, if the electronic device still detects an ECG by using the frequency domain bandwidth of 0.05 Hz to 150 Hz, accuracy of the detected ECG is low (because some of electrical signals within the frequency domain bandwidth of 0.05 Hz to 150 Hz are unavailable), and consequently accuracy of evaluating a health status of the human body is affected. In the conventional technology, a frequency bandwidth of an electronic device configured to detect an ECG is fixed, and the frequency bandwidth is set to 0.05 Hz to 150 Hz. The electronic device that uses the fixed frequency domain bandwidth cannot take into account an electrical signal change of a user in different movement statuses. For example, when the user is in a moving state, the electronic device that uses the fixed frequency domain bandwidth (0.05 Hz to 150 Hz) cannot detect an accurate ECG, and then cannot accurately evaluate a health status of the user. To resolve this technical problem, in an ECG detection solution provided in an embodiment of this application, an electronic device may flexibly set a frequency bandwidth, for example, may collect ECGs by using different frequency bandwidths based on different movement statuses of a user. Regardless of a movement status of the user, a relatively accurate ECG of the user in the movement status can be obtained, thereby improving accuracy of evaluating a health status of the user.

An ECG detection method provided in an embodiment of this application may be applied to an electronic device. The electronic device may be a wearable electronic device (also referred to as a wearable device), such as a watch, a wristband, a headset, or a helmet (such as a virtual reality helmet), or may be a non-wearable device, for example, a portable electronic device with an ECG detection function, such as a mobile phone, a tablet computer, or a notebook computer. An example embodiment of the portable electronic device includes but is not limited to a portable electronic device using iOS^{®}, Android^{®}, Microsoft^{®}, or another operating system. It should be understood that the electronic device may not be a portable electronic device, but is a desktop computer that can detect an ECG. This is not limited in this embodiment of this application. The following embodiments of this application use an example in which an electronic device is a wearable device.

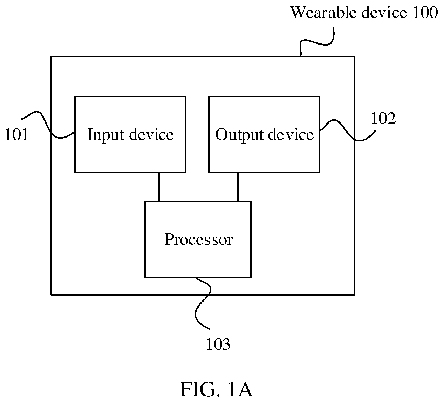

FIG. 1A is a functional block diagram of a wearable device according to an embodiment of this application. In some embodiments, a wearable device 100 may be a watch, a smart band, or the like. As shown in FIG. 1A, the wearable device 100 may include one or more input devices 101, one or more output devices 102, and one or more processors 103. The input device 101 may detect various types of input signals (which may be inputs for short), and the output device 102 may provide various types of output information (which may be outputs for short). The processor 103 may receive an input signal from the one or more input devices 101, generate output information in response to the input signal, and output the output information by using the one or more output devices 102.

In some embodiments, one or more input devices 101 may detect various types of inputs and provide signals (for example, input signals) corresponding to the detected inputs; and then the one or more input devices 101 may provide the input signals for the one or more processors 103. In some examples, the one or more input devices 101 may include any part or component that can detect an input signal. For example, the input device 101 may include an audio sensor (such as a microphone), an optical or visual sensor (such as a camera, a visible light sensor, or an invisible light sensor), a proximity sensor, a touch sensor, a pressure sensor, a mechanical device (such as a crown, a switch, a button, or a key), a vibration sensor, a motion sensor (also referred to as an inertial sensor, such as a gyroscope, an accelerometer, or a speed sensor), a position sensor (such as a global positioning system (GPS)), a temperature sensor, a communications device (such as a wired or wireless communications apparatus), or an electrode. Alternatively, the input device 101 may be a combination of some of the foregoing various parts.

In some embodiments, the one or more output devices 102 may provide various types of outputs. For example, the one or more output devices 102 may receive one or more signals (for example, output signals provided by the one or more processors 103) and provide outputs corresponding to the signals. In some examples, the output device 102 may include any appropriate part or component configured to provide an output. For example, the output device 102 may include an audio output device (such as a speaker), a visual output device (such as a lamp or a display), a tactile output device, or a communications device (such as a wired or wireless communications device). Alternatively, the output device 102 may be a combination of some of the foregoing various parts.

In some embodiments, the one or more processors 103 may be coupled to the input device 101 and the output device 102. The processor 103 may communicate with the input device 101 and the output device 102. For example, the one or more processors 103 may receive an input signal (for example, an input signal corresponding to an input detected by the input device 101) from the input device 101. The one or more processors 103 may parse the received input signal to determine whether to provide one or more corresponding outputs in response to the input signal. If yes, the one or more processors 103 may send an output signal to the output device 102 to provide an output.

In some embodiments, the one or more input devices 101 may further include a group of electrodes (which may be an electrode group for short), and the group of electrodes may include at least two electrodes. The electrode group may be disposed on one or more outer surfaces of the wearable device 100. The one or more processors 103 may monitor voltages or signals received by the electrode group. In some embodiments, the electrode may be configured to provide an ECG function for the wearable device 100. For example, when a user is in contact with a first electrode and a second electrode on the wearable device 100, the wearable device 100 may provide a two-lead ECG function, that is, the wearable device 100 may obtain an ECG signal based on a first electrical signal detected by the first electrode and a second electrical signal detected by the second electrode. For another example, when a user is in contact with a first electrode, a second electrode, and a third electrode on the wearable device 100, the wearable device 100 may provide a three-lead ECG function, that is, the wearable device 100 may obtain an ECG signal based on a first electrical signal detected by the first electrode, a second electrical signal detected by the second electrode, and a third electrical signal detected by the third electrode. Usually, more electrodes indicate that more electrical signals are collected and a more accurate ECG is obtained. The following embodiments of this application mainly use an example in which the wearable device 100 includes two electrodes.

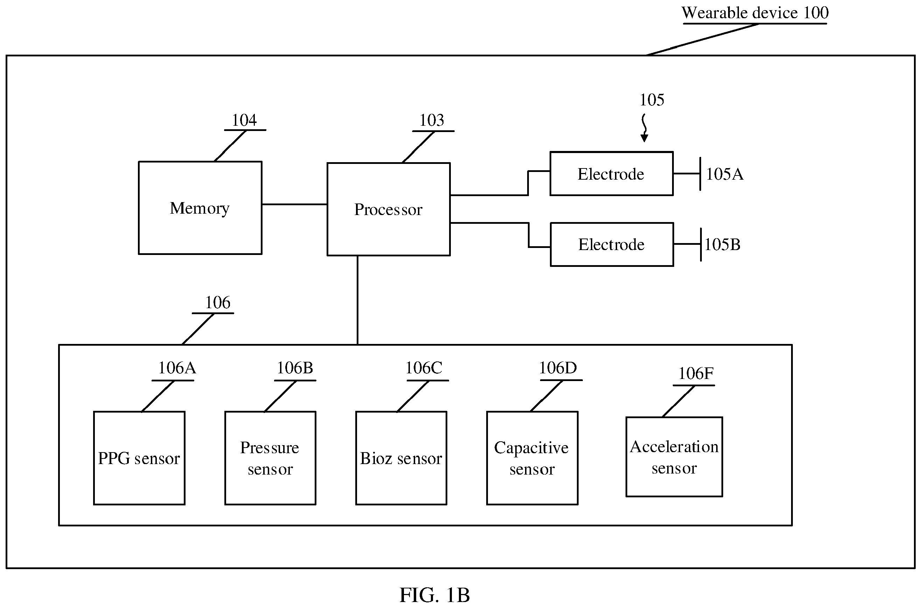

FIG. 1B is a functional block diagram of a wearable device 100 according to another embodiment of this application. In some embodiments, the wearable device 100 may be a watch, a smart band, or the like. As shown in FIG. 1B, the wearable device 100 includes a processor 103, a memory 104, electrodes 105, and a sensor module 106. It may be understood that the parts shown in FIG. 1B constitute no specific limitation on the wearable device 100. The wearable device 100 may alternatively include more or fewer parts than those shown in the figure, combine some parts, split some parts, or have different part arrangements.

The processor 103 may include one or more processing units. For example, the processor 103 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and/or a neural-network processing unit (neural-network processing unit, NPU). Different processing units may be independent components, or may be integrated into one or more processors. The controller may be a nerve center and a command center of the wearable device 100. The controller may generate an operation control signal based on an instruction operation code and a time sequence signal, to complete control of instruction reading and instruction execution. In some other embodiments, a memory may be further disposed in the processor 103, and is configured to store instructions and data. **In** some embodiments, the memory in the processor 103 is a cache. The memory may store instructions or data that has been used or cyclically used by the processor 103. If the processor 103 needs to use the instructions or the data again, the processor 103 may directly invoke the instructions or the data from the memory, to avoid repeated access and reduce a waiting time of the processor 110, thereby improving system efficiency. The processor 103 may run software code/modules of an ECG detection method provided in some embodiments of this application, to detect a physiological parameter of a user, such as an electrocardiogram ECG, an atrial fibrillation indicator, or a heart rate indicator of the user. The following embodiments use the ECG as an example.

The processor 103 may be configured to process an electrical signal (for example, an electrical signal of a human body) detected by the electrode 105 (for example, an electrode 105A or an electrode 105B) into an ECG. For example, the processor 103 may internally include one or more filters, or the processor 103 may be connected to one or more filters. The one or more filters may be configured to filter the electrical signal (for example, the electrical signal of the human body) detected by the electrode 105. For example, the processor 103 may configure frequency domain bandwidths of the one or more filters. If a frequency bandwidth of a filter is 0.5 Hz to 40 Hz, the filter may filter input signals (for example, electrical signals detected by the electrodes 105) of the filter to obtain an electrical signal within the range of 0.5 Hz to 40 Hz, and an electrical signal of another frequency is filtered out. In some embodiments, the function of processing an electrical signal detected by the electrode 105 into an ECG may be performed by another part, component, or circuit, and the another part, component, or circuit may be a different part independent of the processor 103. The another part, component, or circuit may be built up by using separate devices (for example, semiconductor devices). For example, the another part, component, or circuit may be an integrated circuit (integrated circuit, IC), a microcircuit (microcircuit), a chip (chip), or a microchip (microchip) that integrates an ECG detection function. This is not limited in this embodiment of this application.

The sensor module 106 may include a photoplethysmogram (photo plethysmo graphy, PPG) sensor 106A, a pressure sensor 106B, a bioimpedance zinvasion (bioimpedance zinvasion, Bio-z) sensor 106C, a capacitive sensor 106D, an acceleration sensor 106F, and the like. It should be understood that FIG. 1B shows only an example in which several types of sensors are listed. In an actual application, the wearable device 100 may alternatively include more or fewer sensors, replace the listed sensor with another sensor that has a same or similar function, or the like. This is not limited in this embodiment of this application.

The PPG sensor 106A may be configured to detect a heart rate, that is, a quantity of heartbeats in unit time. The PPG sensor 106A may include a light emitting part and a light receiving part. The light emitting part may irradiate a light beam into the human body (for example, a blood vessel), the light beam is reflected/refracted in the human body, and reflected/refracted light is received by the light receiving part to obtain an optical signal. Because transmittance changes in a blood fluctuation process, reflected/refracted light changes, and an optical signal detected by the PPG sensor 106A also changes. The PPG sensor 106A may convert the optical signal into an electrical signal, and determine a heart rate corresponding to the electrical signal.

The pressure sensor 106B may be configured to detect a value of pressure between the human body and the wearable device 100. The pressure sensor 106B is configured to sense a pressure signal, and can convert the pressure signal into an electrical signal. When the pressure sensor 106B is disposed on the electrode 105A and/or the electrode 105B, a greater pressure signal detected by the pressure sensor 106B indicates a stronger electrical signal, which indicates greater pressure (touch intensity or touch strength) between the user and the electrode 105A and/or the electrode 105B. There are many types of pressure sensors 106B, such as a resistive pressure sensor, an inductive pressure sensor, and a capacitive pressure sensor. This is not limited in this embodiment of this application.

The Bio-z sensor 106C may be configured to detect a bioimpedance value of the human body. When the Bio-z sensor 106C is disposed on the electrode 105A and/or the electrode 105B, the Bio-z sensor 106C may detect a bioimpedance value of a human body portion in contact with the electrode 105A and/or the electrode 105B. The bioimpedance value detected by the Bio-z sensor 106C may reflect a skin status of the human body, such as moist skin, dry skin, whether skin has a stain, or whether skin has a thick cuticle. For example, when the Bio-z sensor 106C detects a relatively large bioimpedance value, it indicates that the skin of the human body is dry, for example, the skin has a stain or a thick cuticle; or when the Bio-z sensor 106C detects a relatively small bioimpedance value, it indicates that the skin of the human body is moist, for example, sweats or has a water stain. Different skin statuses of the human body affect an electrical signal generated on a surface of the human body, and then affect accuracy of obtaining the ECG by the wearable device 100. Therefore, when generating the ECG, the wearable device 100 may make reference to the bioimpedance value detected by the Bio-z sensor 106C. Specific content is described below.

The capacitive sensor 106D may be configured to detect a capacitance between the human body and the wearable device 100, and the capacitance may reflect whether the human body is in good contact with the electronic device. When the capacitive sensor 106D is disposed on the electrode 105A and/or the electrode 105B, the capacitive sensor 106D may detect a capacitance between the human body and the electrode 105A and/or the electrode 105B. When the capacitive sensor 105D detects a quite large or quite small capacitance, it indicates that the human body is in relatively poor contact with the electrode 105A and/or the electrode 105B; or when the capacitive sensor 106D detects a moderate capacitance, it indicates that the human body is in relatively good contact with the electrode 105A and/or the electrode 105B. Whether the human body is in good contact with the electrode affects electrical signal detection of the electrode, and then affects ECG generation. Therefore, when generating the ECG, the wearable device 100 may make reference to the capacitance detected by the capacitive sensor 106D. Specific content is described below.

The acceleration sensor 106F may be configured to detect acceleration values of the wearable device 100 in all directions (usually on three axes). The wearable device 100 is a wearable device. When the user wears the wearable device 100, the wearable device 100 is driven by the user to move. Therefore, the acceleration values that are in all the directions and that are detected by the acceleration sensor 106F may reflect a movement status of the human body.

The memory 104 may be configured to store computer-executable program code, where the executable-program code includes instructions. The processor 103 runs the instructions stored in the memory, to perform various function applications and data processing of the wearable device 100. The memory may include a high-speed random access memory, or may include a nonvolatile memory, such as at least one magnetic disk memory, flash memory, or universal flash storage (universal flash storage, UFS). This is not limited in this embodiment of this application. In some embodiments, the wearable device 100 may store an obtained ECG signal in the memory 104, for ease of viewing by the user.

In some embodiments, the wearable device 100 may include a display (or a display screen), or may not include a display. For example, when the wearable device 100 is a wristband, the wearable device 100 may include a display, or may not include a display; or when the wearable device 100 is a watch, the wearable device 100 may include a display. The display may be configured to display an ECG, health status information, a display interface of another application, or the like. The display includes a display panel. The display panel may be a liquid crystal display (liquid crystal display, LCD), an organic light-emitting diode (organic light-emitting diode, OLED), an active-matrix organic light emitting diode (active-matrix organic light emitting diode, AMOLED), a flexible light-emitting diode (flex light-emitting diode, FLED), a mini-LED, a micro-LED, a micro-OLED, quantum dot light emitting diodes (quantum dot light emitting diodes, QLED), or the like. In some embodiments, a touch sensor may be disposed on the display to form a touchscreen. This is not limited in this embodiment of this application. The touch sensor is configured to detect a touch operation performed on or near the touch sensor. The touch sensor may transmit the detected touch operation to the processor 103, to determine a touch event type. A visual output related to the touch operation may be provided by using the display.

In some embodiments, the wearable device 100 may have a communication function, or may not have a communication function. For example, the wearable device 100 may send, by using a communications module, a collected ECG signal to a network side or another device, such as a mobile phone, connected to the wearable device 100, so that the user views and stores the ECG in the mobile phone. In some embodiments, the wearable device 100 may include a wireless communications module and/or a mobile communications module, and one or more antennas. The wearable device 100 may implement the communication function by using the one or more antennas, and the wireless communications module or the mobile communications module. In some examples, the mobile communications module may provide a solution to wireless communication that includes 2G/3G/4G/5G and that is applied to the wearable device 100. The wireless communications module may provide a solution, applied to the wearable device 100, to wireless communication including a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like. The one or more antennas may be configured to transmit and receive electromagnetic wave signals.

In some embodiments, the mobile communications module may be coupled to the one or more antennas. For example, the mobile communications module may receive an electromagnetic wave by using the one or more antennas, perform processing such as filtering and amplification on the received electromagnetic wave to obtain an electrical signal, and transmit the electrical signal to the processor 103 for processing (for example, the processor 103 determines whether to provide a corresponding output in response to the electrical signal). The mobile communications module may further amplify a signal obtained by the processor 103 after the processing, and a signal obtained after the amplification is radiated after being converted into an electromagnetic wave by using the one or more antennas. In some other embodiments, the wireless communications module may also be coupled to the one or more antennas. For example, the wireless communications module may receive an electromagnetic wave by using the one or more antennas, perform processing such as filtering and amplification on the received electromagnetic wave, and send a signal obtained after the processing to the processor 103 for processing. The wireless communications module may further amplify a signal obtained by the processor 103 after the processing, and a signal obtained after the amplification is radiated after being converted into an electromagnetic wave by using one or more antennas.

In some embodiments, the wearable device 100 may further include a power supply module, such as a battery, to supply power to parts such as the processor 103, a first electrode, a second electrode, and the sensor module 106 in the wearable device 100. In some other embodiments, the wearable device 100 may be further connected to a charging device (in, for example, a wireless or wired manner), and the power supply module may receive electric energy that is input by the charging device, to store power for the battery.

The electrodes 105 may include at least two electrodes (an electrode group for short). In FIG. 1B, two electrodes (the electrode 105A and the electrode 105B) are used as an example. When a body portion of the user is in contact with the electrodes 105, the human body and the wearable device 100 form a conductive loop by using the two electrodes. In a heart beating process of the human body, an electric charge in the body generates an electrical signal, and the electrode 103 may capture an electrical signal of the skin of the human body. One or more of the electrodes 105 may be made of a conductive material (referred to as an electrode material below), and the conductive material is any material with conductivity, such as a metal material (such as copper, aluminum, iron, cobalt, or nickel), an alloy material (such as a chromium copper alloy), a metal oxide (such as aluminum copper oxide), or composite metal; or may be made of stainless steel (SUS) or diamond-like carbon (DLC), or include stainless steel (SUS), diamond-like carbon (DLC), or the like. This is not limited in this embodiment of this application.

In some embodiments, a filtering apparatus (such as a filter, not shown) may be disposed between the processor 103 and the electrode group. The filtering apparatus may filter electrical signals detected by the electrode group. For example, after collecting electrical signals, the electrode 105A and the electrode 105B may provide the electrical signals for the filtering apparatus. The filtering apparatus may filter, by using a specific frequency bandwidth, the electrical signals provided by the electrode 105A and the electrode 105B. For example, frequencies of the electrical signals detected by the electrode group fall within a range of 0.05 Hz to 150 Hz. The filtering apparatus is configured to filter, by using a frequency bandwidth of 7 Hz to 23 Hz, the electrical signals of the human body that are collected by the electrode group. Therefore, a frequency of an electrical signal retained after the filtering falls within the range of 7 Hz to 23 Hz, and an electrical signal outside the frequency bandwidth of 7 Hz to 23 Hz is filtered out. The filtering apparatus provides the retained electrical signal for the processor 103, and the processor 103 obtains a physiological parameter, such as an ECG signal, of the human body, based on the electrical signal. In some other embodiments, a filtering function of the filtering apparatus may be alternatively integrated into the processor 103, that is, the processor 103 filters the electrical signals collected by the electrode group, and then obtains an ECG based on an electrical signal retained after the filtering.

In some embodiments, the processor 103 may determine an appropriate frequency bandwidth based on a current status of the user, and then configure the frequency bandwidth for the filtering apparatus. The filtering apparatus filters, by using the frequency bandwidth, the electrical signals collected by the electrode group. The current status of the user may include a current movement status, skin status, or the like of the user. For example, the processor 103 may determine the current status of the user by using sensor data detected by the sensor module 106, and then select an appropriate frequency bandwidth based on different sensor data. Specific content is described below.

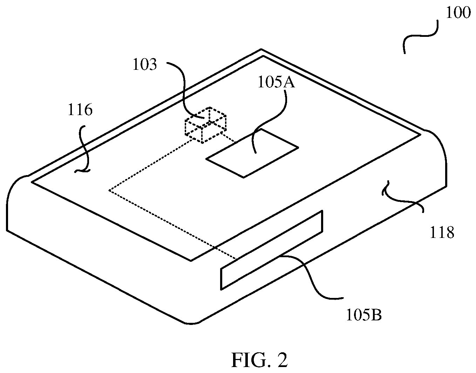

FIG. 2 shows an example of a wearable device 100 according to an embodiment of this application. The wearable device 100 may be a watch or a wristband. For parts or components in the wearable device 100, refer to the descriptions in FIG. 1A and FIG. 1B. A group of electrodes (an electrode group for short) may be disposed on the wearable device 100. For example, the electrode group may include an electrode 105A and an electrode 105B. In some embodiments, electrodes in the electrode group may be disposed on one surface of the wearable device 100. In some other embodiments, electrodes in the electrode group may be disposed on different surfaces of the wearable device 100, and the electrodes disposed on the different surfaces of the wearable device 100 may facilitate a user in enabling different body portions to be in contact with different electrodes. For example, referring to FIG. 2, the electrode group may include a first electrode 105A disposed on a first surface 116 of the wearable device 100 and a second electrode 105B disposed on a second surface 118 of the wearable device 100. The user may enable a body portion to be in contact with one or more electrodes (for example, the first electrode 105A) on the wearable device 100, and enable another body portion to touch one or more other electrodes (for example, the second electrode 105B). The first electrode and the second electrode may detect electrical signals of a human body. A processor 103 in the wearable device 100 or a processor in another device (such as a mobile phone) connected to the wearable device 100 may determine a physiological parameter of the user, such as an electrocardiogram (ECG) of the user, based on the electrical signals detected by the first electrode and the second electrode. In some embodiments, the electrode group may alternatively include more electrodes, for example, may include a third electrode in addition to the first electrode 105A and the second electrode 105B. The third electrode may be disposed on a different surface from the first electrode and the second electrode. For example, the third electrode is disposed on a surface opposite to the first surface 116, that is, a lower surface. Alternatively, the third electrode and the first electrode 105A are disposed at different positions on the first surface 116, or the third electrode and the second electrode 105B are disposed at different positions on the second surface 118.

In some embodiments, to improve a light and thin sense of the wearable device 100, a thickness of the electrode may be set to be relatively small. For example, a PVD coating method (which may also be referred to as a PVD deposition method) may be used to coat an outer surface of the wearable device 100 with a layer of film material (such as one or more of the electrode materials listed above) to form an electrode. For example, in FIG. 2, the electrode may be disposed on the outer surface of the wearable device 100 through PVD deposition. The outer surface may be any transparent, translucent, or opaque surface made of an amorphous solid, such as glass, a crystal or crystalline material (such as sapphire or zirconia), or plastic.

In some embodiments, the electrode may be disposed on an outer surface of a housing of the wearable device 100 through PVD deposition, or may be disposed on a carrier on the housing of the wearable device 100 through PVD deposition. In some embodiments, the carrier may be capable of supporting any appropriate structure of the electrode, and the electrode may be "formed or attached" onto the carrier. In some embodiments, the carrier may be an optically transparent material in any shape, or may be made of different materials, including an opaque material. In some embodiments, the carrier may be in any shape. If the electrode is disposed on the outer surface of the housing of the wearable device 100 through PVD deposition, an electrical contact point may be disposed in an area covered by the electrode on the outer surface (for example, the area covered by the electrode on the outer surface is a conductor), and the electrode may be connected to a part (for example, the processor 103) inside the wearable device 100 by using the electrical contact point. If the electrode is disposed on the carrier on the housing of the wearable device 100 through PVD deposition, an edge or a periphery of the carrier may be coated with an electrode material. For example, the electrode may be formed on an outer surface of the carrier, an electrical contact point is disposed on the outer surface, and the electrical contact point may be connected to another carrier that includes (is filled or coated with) a conductive material. The another carrier may be connected to another part (such as the processor 103) in the wearable device 100 by using a through hole in the housing.

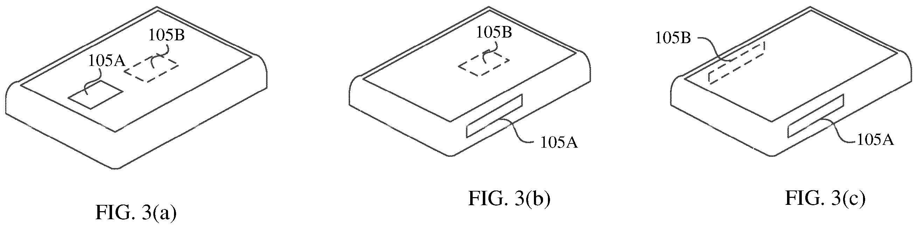

FIG. 3(a) to FIG. 3(c) show some other examples of a wearable device 100 according to an embodiment of this application. The wearable device 100 may be a watch or a wristband. As shown in FIG. 3(a), an electrode 105A is disposed on an upper surface (opposite to a lower surface, where the lower surface is a surface that is of the wristband and that is in contact with a wrist of a human body) of the wearable device 100; and an electrode 105B is disposed on the lower surface of the wearable device 100, and therefore is represented by dashed lines in the figure. For example, assuming that a left wrist of a user wears the wearable device 100 shown in FIG. 3(a), the left wrist is in contact with the electrode 105B on the lower surface of the wearable device 100, and the user may be in contact with the electrode 105A on the upper surface with a right hand finger, to implement ECG detection. It should be understood that, assuming that a right wrist of the user wears the wearable device 100 shown in FIG. 3(a), the right wrist may be in contact with the electrode 105B on the lower surface of the wearable device 100, and the user may be in contact with the electrode 105A on the upper surface with a left hand finger, to implement ECG detection. Referring to FIG. 3(b), an electrode 105A may be disposed on a side surface of the wearable device 100, and an electrode 105B is disposed on a lower surface of the wearable device 100 (shown by dashed lines in the figure). Referring to FIG. 3(c), an electrode 105A and an electrode 105B are separately disposed on different side surfaces. Assuming that a left wrist of a user wears the wearable device 100 shown in FIG. 3(c), two fingers (for example, a thumb and an index finger, or an index finger and a middle finger) of a right hand of the user may be respectively in contact with the electrode 105A and the electrode 105B, to implement ECG detection.

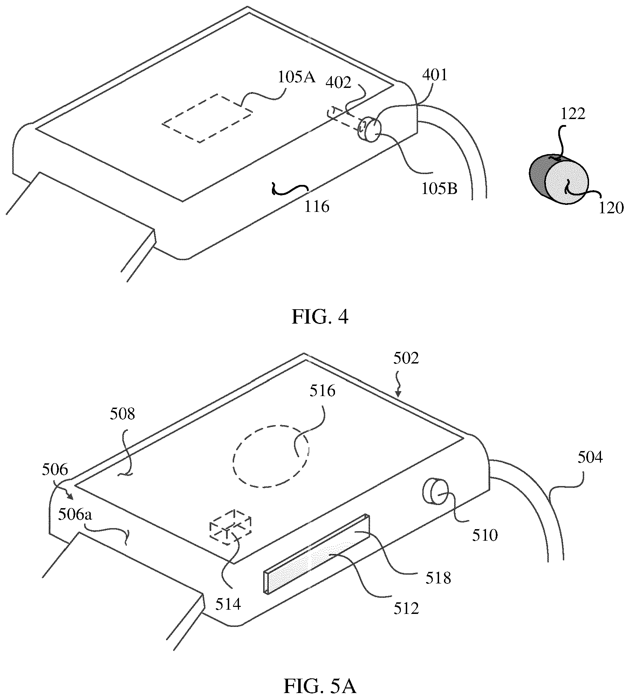

FIG. 4 shows another example of a wearable device 100 according to an embodiment of this application. In some embodiments, the wearable device 100 may be a watch. An electrode group may include a first electrode 105A disposed on a lower surface and a second electrode 105B disposed on a crown 401. In some embodiments, the second electrode 105B may be disposed on an upper surface 120 of the crown 401, or on a side surface 122 of the crown 401. A lower surface opposite to the upper surface 120 is a surface in contact with a side surface 116 of the wearable device 100. The crown 401 may be made of a conductive material, or may have a conductive surface. A conductive portion of the crown 401 may be connected to a conductive shaft 402 (for example, a rotatable shaft), and the shaft 402 extends to an inner portion of a housing through an opening in the housing. The electrode 105B may be connected to another part (such as a processor 103) inside the wearable device 100 by using the conductive portion in the crown 401 and the shaft 402. In some embodiments, a processor (for example, the processor 103) of the wearable device 100 may be configured to determine a physiological parameter of a user based on electrical signals detected by various electrodes (for example, the electrode 105A and the electrode 105B). In some embodiments, the physiological parameter may include an ECG of the user. For example, in the wearable device 100 shown in FIG. 4, an outer surface (for example, the lower surface) of the wearable device 100 may have the first electrode 105A, and the crown 401 may have the second electrode 105B. When the user fastens the wearable device 100 to a wrist of the user, the first electrode 105A may be in contact with skin of the wrist of the user. To obtain an ECG, the user may touch the second electrode 105B on the crown 401 with a finger of the other hand of the user. In some other embodiments, more electrodes are disposed on the wearable device 100. For example, the wearable device 100 further includes a third electrode in addition to the first electrode 105A and the second electrode 105B. For example, the third electrode may be disposed on an upper surface of the wearable device 100. In this case, when the user is in contact with the first electrode and the second electrode, the wearable device 100 may obtain an ECG by using a first electrical signal detected by the first electrode and a second electrical signal detected by the second electrode; or when the user is in contact with the first electrode, the second electrode, and the third electrode, the wearable device 100 may obtain an ECG by using a first electrical signal detected by the first electrode, a second electrical signal detected by the second electrode, and a third electrical signal detected by the third electrode.

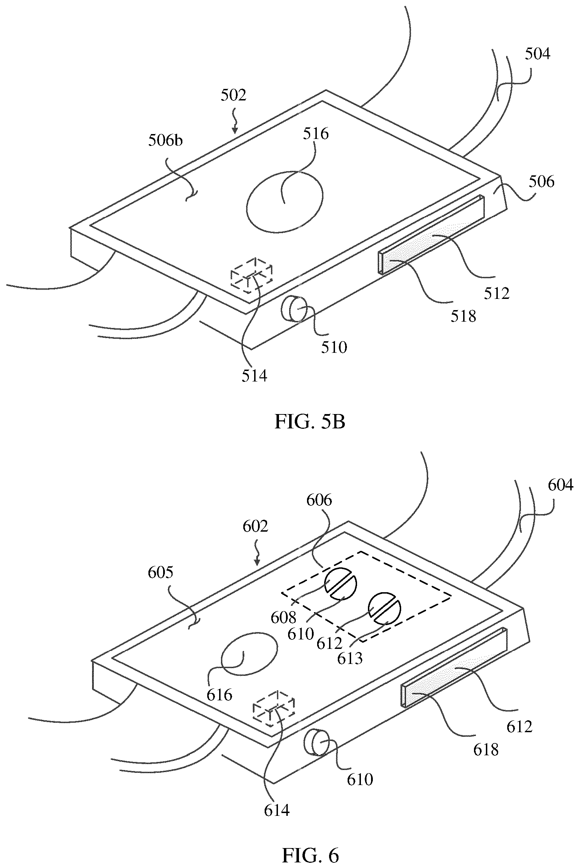

FIG. 5A and 5B show examples of a watch 500 according to an embodiment of this application. For the watch 500, refer to the examples of the wearable device 100 that are described in FIG. 1A to FIG. 4. As shown in FIG. 5A, the watch 500 may include a watch body 502 and a watchband 504. The watch body 502 may include an input device, such as a crown (crown) 510 or a button 512. It should be understood that in FIG. 5A and FIG. 5B, only a portion of the watchband 504 is shown (that is, only a portion that is of the watchband 504 and that is connected to the watch body 502 is shown).

The watch body 502 may include a housing 506. The housing 506 may include a single housing member or more than two housing members. For example, the housing 506 may include a front-side housing member 506a, and when the watch 500 is worn by a user, the front-side housing member 506a is opposite to skin of the user (referring to FIG. 5A). The housing 506 may further include a rear-side housing member 506b (or a rear cover), that is, a housing member that faces the skin of the user (referring to FIG. 5B). One or more housing members may be metal, plastic, ceramic, crystal, another-type housing members, or a combination of these materials.

As shown in FIG. 5A, an opening may be disposed in the housing 506 (for example, the front-side housing member 506a), and a cover 508 may be attached into or onto the opening. The cover 508 may be located on a display in the housing 506. In some embodiments, the cover 508 may be transparent. The user can see the display through the cover 508. In some embodiments, the display may display an ECG waveform of the user that wears or otherwise uses the watch 500, or the display may display information such as time or an icon. In some examples, the cover 508 may include a crystal, such as a sapphire crystal, or the cover 508 may be made of glass, plastic, or another material. In some embodiments, an outer surface of the cover 508 may be used as an input receiving apparatus (that is, used as an input apparatus) and an output providing apparatus (that is, used as an output apparatus). For example, the cover 508 may have a touch sensing capability or a pressure sensing capability. For example, a touch sensor or a pressure sensor is disposed on the cover 508. The user may interact with the watch 500 on the cover 508. As an example, the user may touch or press a position of an image on the cover 508 to choose to (or otherwise) open or edit the image. In some embodiments, the cover 508 may be a touch display.

In some embodiments, the watch body 502 may include at least one input device, such as the crown 510 and the button 512, or another input device, such as a scroll wheel or a dial. The user of the watch 500 may operate the input device to implement a corresponding function. For example, for the crown 410, the user may rotate, translate, tilt, or be in contact with the crown 410 to implement an operation such as startup or determining. Certainly, the user may alternatively implement an operation such as startup or determining by using the cover 508 (for example, a touch display).

As shown in FIG. 5B, the housing 506 may include a structure configured to attach the watchband 504 onto the body 502. In some cases, the structure may include a groove or an opening, and an end portion of the watchband 504 may be inserted into and connected to the watch body 502 through the groove or the opening. The watchband 504 may be configured to fasten the watch 500 to the user. In some embodiments, the watch 500 may include a group of electrodes. As shown in FIG. 5A and FIG. 5B, the electrode group may include a first electrode 516 disposed on the rear-side housing member 506b and a second electrode 518 disposed on the crown 510 and the button 512. The first electrode 516 and the second electrode 518 may be connected to an internal processor 514 to sense a physiological parameter (such as an ECG) of a user that wears the watch 500 or a user otherwise in contact with the first electrode and the second electrode.

In some embodiments, the electrode 516 may be formed on the rear-side housing member 206b (through, for example, PVD deposition or electroplating). If the rear-side housing member 506b is not conductive, the electrode 516 may be directly formed on the rear-side housing member 506b, and is connected to a circuit (such as the processor 514) inside the watch body 502 through a through hole. The through hole may penetrate through the rear-side housing member 506b. If the rear-side housing member 506b is conductive, the electrode 516 may be separated from the rear-side housing member 506b by using an insulator or an insulation layer. For example, a protrusion insulated from the rear-side housing member 206b is disposed on the rear-side housing member 206b, and the electrode 516 may be attached onto the protrusion. The protrusion may internally include a conductor, an outer surface of the conductor is separated from the rear-side housing member 506b by using an insulator, the electrode is formed on the protrusion, and the electrode may be connected to another part (such as the processor 514) inside the watch 500 by using the conductor inside the protrusion.

In some embodiments, the electrode 518 on the crown 510 or the button 512 may be a conductive surface of the crown 510 or the button 512. For example, an entire outer surface of the crown 510 or the button 512 is a conductor, and the conductor may act as the electrode 512. For another example, the crown 510 or the button 512 may have a conductive portion (such as core or insert), and the electrode 518 is formed on an outer surface of the crown 510 or the button 512. For example, when the front-side housing member 506a is conductive, the crown 510 or the button 512 (or the conductive part thereof) may be insulated from the front-side housing member 506a by using an insulator, and the electrode 518 may be formed on the outer surface of the crown 510 or the button 512 and connected to another part (such as the processor 514) inside the watch 500 by using the conductive portion in the crown 510 or the button 512.

In some embodiments, the watch 500 may have no display, crown 510, or button 512. For example, the watch 500 may include an audio input or output interface, a touch input interface, a tactile (force) input or output interface, or another input or output interface that does not need the display, the crown 510, or the button 512. When the watch 500 has no display, a front surface of the watch 500 may be covered by the cover 508, or may be covered by metal or another-type housing member. In these embodiments, the electrode 518 on the crown 510 or the button 512 may be replaced with an electrode on the front surface of the watch body 502, or an electrode is added on the front surface of the watch body 502 in addition to the electrode 518 on the crown 510 or the button 512 and the electrode 516 on a back surface. The user may touch the electrode on the front surface with a finger. Specifically, the user may determine, based on a preference, to touch a specific electrode on the watch 500.

FIG. 6 shows another example of a digital watch 600 according to an embodiment of this application. For the watch 600, refer to the examples of the wearable device 100 that are described in FIG. 1A to FIG. 4. The watch 600 may include a watch body 602 and a watchband 604. It should be understood that FIG. 6 shows only a portion of the watchband 604 (that is, only a portion that is of the watchband 604 and that is attached to the watch body 602). The watch 600 in FIG. 6 includes more components than the watch 500 shown in FIG. 5A and FIG. 5B. For example, more parts are provided or exposed on a rear-side housing member 605 of the watch 600. For example, the rear-side housing member 605 of the watch 600 may include an electrode 616. In some embodiments, the electrode 616 may be circular and may be disposed on the rear-side housing member 605 through PVD deposition. The rear-side housing member 605 may further include a sensor subsystem 606, and the sensor subsystem 606 may include a temperature sensor, a Bio-z sensor, a PPG sensor, and the like. Certainly, the sensor subsystem 606 may further include another sensor, such as an accelerometer. For example, for the PPG sensor, the PPG sensor may include an optical component. The optical component may include one or more windows 608, 610, 612, and 613 in the rear-side housing member 605. Light of at least one wavelength can pass through each of the windows 608, 610, 612, and 613. In some cases, each of the windows 608, 610, 612, and 613 may be semicircular, or in another shape. The window may be made of a crystal, glass, plastic, or another material, and the window can transmit light that is of at least one wavelength and that is emitted or received by the sensor subsystem 606.

As an example, FIG. 6 shows the windows 608 and 610 and the windows 612 and 613 that are aligned along a first axis, and the first axis may divide the rear housing member 602 into two halves (for example, two halves with equal areas). In some embodiments, each pair of windows 608/610, or windows 612/613 that form a circular area may include a first window and a second window, one or more light emitters are placed below the first window, and one or more light receivers are placed below the second window. One or more light blocking walls are disposed between each pair of windows. For example, the light blocking wall may be located between one or more light emitters and one or more light receivers to isolate emitted light and received light from each other. In some embodiments, for example, for the pair of windows 608/610, one or more light emitters are disposed below the window 608, and one or more light receivers are disposed below the window 610. For example, light emitted by the one or more light emitters below the window 608 may be irradiated into a human body (such as a blood vessel of the human body), and light internally refracted or reflected by the human body may be captured by the one or more light receivers below the window 610. The light emitter may be a visible light or an invisible light emitter. The light receiver may be connected to a processor 614. For example, the light receiver may convert a received optical signal into an electrical signal, and then generate a physiological parameter, such as a heart rate, based on the electrical signal.

In some embodiments, the rear-side housing member 605 may include a transparent cover (for example, a cover that includes a crystal such as a sapphire crystal, a glass cover, or a plastic cover); and may be flat or planar (as shown in the figure), or may be curved or non-planar. In some other embodiments, the rear-side housing member 605 may be an opaque substrate, such as a metal or plastic substrate, and the one or more windows 608, 610, 612, and 613 (for example, transparent windows) may be assembled to an opening in the substrate. The electrode 616 may be installed in another opening. In some embodiments, the electrode 616 or the one or more windows 608, 610, 612, and 613 may be formed on a protrusion protruding from an outer surface of the rear-side housing member 605 (a material of the protrusion may be different from a material of the rear-side housing member 605). This is not limited in this embodiment of this application.

In another embodiment, the wearable device 100 may be another device, such as a headset. An electrode group may be disposed on different surfaces of the headset, to implement ECG detection. The headset may be a wired headset or a wireless headset (such as a Bluetooth headset).

In the following embodiments of this application, the watch 600 shown in FIG. 6 is used as an example. In some embodiments, the first electrode 616 is disposed on a lower surface of the watch 600, and a second electrode 618 is disposed on a button 612. Assuming that a left wrist of a user wears the watch 600, the left wrist of the user may be in contact with the first electrode 616 on the watch 600, and another body portion (such as a right hand finger) of the user may be in contact with the second electrode 618 on the button 612. The first electrode 616 detects a first electrical signal. The second electrode 618 detects a second electrical signal. Both the first electrical signal and the second electrical signal have specific frequencies. The watch 600 may obtain a physiological parameter of the user, such as an ECG of the user, by using the detected first electrical signal and second electrical signal. The following uses the ECG of the user as an example.

In some embodiments, the watch 600 may enable an automatic ECG detection function. After the watch 600 enables the automatic ECG detection function, parts in the watch 600 that are related to the ECG detection function, such as the first electrode 616, the second electrode 618, and all or some sensors (for example, sensors related to the ECG detection function, such as a PPG sensor and a motion sensor) in the sensor subsystem 606 are in an enabled state. For example, a power supply module in the watch 600 may continuously supply power to these parts, so that these parts are in the enabled state. A possible implementation in which the watch 600 enables the automatic ECG detection function is described below.

In some embodiments, after the watch 600 enables the automatic ECG detection function, the watch 600 can obtain an ECG provided that a body portion of a user is in contact with the first electrode 616 and the second electrode 618 so that the first electrode 616 and the second electrode 618 detect electrical signals.

In some embodiments, a user may perform various behavioral activities (such as fitness and sleeping) in a process of wearing the watch 600. To improve accuracy of an obtained ECG, when different body portions of the user are in contact with the first electrode 616 and the second electrode 618, the first electrode 616 and the second electrode 618 may detect electrical signals. For example, when a left wrist of the user is in contact with the first electrode 616 and an index finger of a right hand of the user is in contact with the second electrode 618, the watch 600 (such as the processor 614 in the watch 600) may select an appropriate operating mode based on a current status of the user. The watch 600 has different frequency bandwidths (for example, a filtering apparatus in the watch 600 uses different frequency bandwidths when filtering electrical signals collected by the first electrode and the second electrode) in different operating modes. The current status of the user may include a current movement status, skin status, or the like of the user. For example, the watch 600 may determine the current status of the user by using sensor data detected by the sensor subsystem 606, and then select an appropriate frequency bandwidth based on different sensor data. The following embodiment describes an example in which the processor 614 determines an appropriate frequency domain bandwidth based on sensor data.

In some examples, the sensor subsystem 606 may include a Bio-z sensor, that is, a bioimpedance zinvasion sensor. The watch 600 stores a correspondence between an impedance value Z and a frequency domain bandwidth. Table 1 shows an example of the correspondence.

**Table 1**

| Impedance value Z | Frequency domain bandwidth |
|---|---|
| Less than Z1 | 0.5 Hz to 40 Hz |
| Greater than or equal to Z1 and less than or equal to Z2 | 0.05 Hz to 150 Hz |
| Greater than Z2 | 7 Hz to 23 Hz |

As described above, a bioimpedance value detected by the Bio-z sensor may reflect a skin status of a human body. For example, when the Bio-z sensor is disposed on the first electrode or the second electrode, when a body portion of the user is in contact with the first electrode or the second electrode, the Bio-z sensor may detect the skin (for example, skin in contact with the first electrode or the second electrode) status of the user. For example, when the Bio-z sensor detects a relatively large bioimpedance value, it indicates that skin of the human body is dry, for example, the skin has a stain or a thick cuticle; or when the Bio-z sensor detects a relatively small bioimpedance value, it indicates that the skin of the human body is moist, for example, sweats or has a water stain. The stain, cuticle, water stain, or the like on the skin of the human body all affect charge distribution on the skin of the human body, and then affect electrical signals detected by the first electrode and the second electrode. In this case, electrical signals of only a relatively small quantity of frequency bands in all electrical signals detected by the first electrode and the second electrode are available, and an electrical signal of another frequency is noise. Presence of the noise affects ECG generation. Therefore, when determining that an impedance value detected by the Bio-z sensor is less than Z1 (indicating that the skin is quite dry), the processor 614 may determine, based on Table 1, a frequency domain bandwidth corresponding to Z1, that is, 0.5 Hz to 40 Hz; when determining that an impedance value detected by the Bio-z sensor falls within a range from Z1 to Z2, the processor 614 may determine, based on Table 1, a corresponding frequency domain bandwidth, that is, 0.05 Hz to 150 Hz; or when determining that an impedance value detected by the Bio-z sensor is greater than Z2 (indicating that the skin has a water stain), the processor 614 may determine, based on Table 1, a frequency domain bandwidth corresponding to Z2, that is, 7 Hz to 23 Hz.

It should be noted that, a frequency bandwidth corresponding to each impedance value range in this application may be an empirical value determined based on a test. This is not limited in this embodiment of this application. It should be noted that any specific value range (such as 0.05 Hz to 150 Hz, 0.5 Hz to 40 Hz, or 7 Hz to 23 Hz) provided in this specification is merely an example, and is provided to fully describe a principle of this application and an actual application thereof and therefore constitutes no limitation on this application. According to the foregoing instruction content, many modification forms and variation forms, such as a simple modification of a value range, are possible, and all falls within the protection scope of this application.

In some other examples, the sensor subsystem 606 may further include a PPG sensor. The watch 600 (such as a memory) stores a correspondence between a heart rate value and a frequency domain bandwidth. Table 2 shows an example of the correspondence.

**Table 2**

| Collected heart rate | Frequency domain bandwidth |
|---|---|
| Less than a threshold 1 | 0.05 Hz to 150 Hz |
| Greater than or equal to the threshold 1 and less than or equal to a threshold 2 | 0.5 Hz to 40 Hz |
| Greater than the threshold 2 | 7 Hz to 23 Hz |

In some embodiments, the PPG sensor may sense a current heart rate value of the user, and the heart rate value may reflect a current body status of the user. For example, when a heart rate is quite high, it indicates that the user excessively moves. Usually, when the user excessively moves, electrical signals detected by the first electrode and the second electrode are affected. In this case, electrical signals of only a relatively small quantity of frequency bands in all electrical signals detected by the first electrode and the second electrode are available, and an electrical signal of another frequency is noise. Presence of the noise affects ECG generation. Therefore, when determining that a heart rate collected by the PPG sensor is less than the threshold 1 (indicating that the user is in a relatively still state), the processor 614 may determine, based on Table 2, that a frequency domain bandwidth is 0.05 Hz to 150 Hz; when determining that a heart rate collected by the PPG sensor is greater than or equal to the threshold 1 and less than the threshold 2, the processor 614 may determine, based on Table 2, that a frequency domain bandwidth is 0.5 Hz to 40 Hz; or when determining that a heart rate collected by the PPG sensor is greater than the threshold 2 (indicating that the user excessively moves), the processor 614 may determine, based on Table 2, that a frequency domain bandwidth is 7 Hz to 23 Hz.

In some other examples, the sensor subsystem 606 may further include a pressure sensor. The watch 600 (such as a memory) stores a correspondence between pressure and a frequency domain bandwidth. Table 3 shows an example of the correspondence.

**Table 3**

| Pressure value | Frequency domain bandwidth |
|---|---|
| Less than P1 | 0.5 Hz to 40 Hz |
| Greater than or equal to P1 and less than or equal to P2 | 0.05 Hz to 150 Hz |
| Greater than P2 | 7 Hz to 23 Hz |

As described above, the pressure sensor may be configured to sense a pressure signal. For example, when the pressure sensor is disposed on the first electrode or the second electrode, the pressure sensor may detect contact pressure between the body portion of the user and the first electrode or the second electrode. When the pressure sensor detects a greater pressure signal, it indicates greater pressure (touch intensity or touch strength) between the user and the first electrode and/or the second electrode (indicating that the body portion of the user presses the first electrode and/or the second electrode); or when the pressure sensor detects a smaller pressure signal, it indicates smaller pressure (touch intensity or touch strength) between the user and the first electrode and/or the second electrode (indicating that the body portion of the user is in poor contact with the first electrode and/or the second electrode). In these cases, electrical signals of only a relatively small quantity of frequency bands in all electrical signals detected by the first electrode and the second electrode are available, and an electrical signal of another frequency is noise. Presence of the noise affects ECG generation. Therefore, when determining that a pressure value detected by the pressure sensor is less than P1, the processor 614 may determine, based on Table 3, that a corresponding frequency domain bandwidth is 0.05 Hz to 150 Hz; when determining that a pressure value detected by the pressure sensor falls within a range from P1 to P2, the processor 614 may determine, based on Table 3, that a corresponding frequency domain bandwidth is 0.5 Hz to 40 Hz; or when determining that a pressure value detected by the pressure sensor is greater than P2, the processor 614 may determine, based on Table 3, that a corresponding frequency domain bandwidth is 7 Hz to 23 Hz.

In some examples, the sensor subsystem 606 may further include a capacitive sensor. The watch 600 (such as a memory) stores a correspondence between a capacitance and a frequency domain bandwidth. Table 4 shows an example of the correspondence.

**Table 4**

| Capacitance value | Frequency domain bandwidth |
|---|---|
| Less than C1 | 0.5 Hz to 40 Hz |
| Greater than or equal to C1 and less than or equal to C2 | 0.05 Hz to 150 Hz |
| Greater than C2 | 7 Hz to 23 Hz |

As described above, the capacitive sensor may be disposed on the first electrode and/or the second electrode. When the body portion of the user is in contact with the first electrode and/or the second electrode, the capacitive sensor may detect a capacitance between the human body and the first electrode and/or the second electrode. When the capacitive sensor detects a quite large or quite small capacitance, it indicates that the human body is in relatively poor contact with the first electrode and/or the second electrode; or when the capacitive sensor detects a moderate capacitance, it indicates that the human body is in relatively good contact with the first electrode and/or the second electrode. Therefore, when determining that a value detected by the capacitive sensor is less than C1, the processor 614 may determine, based on Table 4, that a corresponding frequency domain bandwidth is 0.5 Hz to 40 Hz; when determining that a value detected by the capacitive sensor is greater than C2, the processor 614 may determine, based on Table 4, that a corresponding frequency domain bandwidth is 7 Hz to 23 Hz; or when determining that a value detected by the capacitive sensor falls within a range from C1 to C2, the processor 614 may determine, based on Table 4, that a corresponding frequency domain bandwidth is 0.05 Hz to 150 Hz.

In some examples, the sensor subsystem 606 may further include an angular velocity sensor. The watch 600 (such as a memory) stores a correspondence between an angular velocity and a frequency domain bandwidth. Table 5 shows an example of the correspondence.

**Table 5**

| Angular velocity | Frequency domain bandwidth |
|---|---|
| Less than A1 | 0.05 Hz to 150 Hz |
| Greater than or equal to A1 and less than or equal to A2 | 0.5 Hz to 40 Hz |
| Greater than A2 | 7 Hz to 23 Hz |

In some embodiments, the angular velocity sensor may reflect a current movement status of the user. Therefore, when determining a value detected by the angular velocity sensor is less than A1, the processor 614 may determine, based on Table 5, that a corresponding frequency domain bandwidth is 0.05 Hz to 150 Hz; when determining that a value detected by the angular velocity sensor is greater than A2, the processor 614 may determine, based on Table 5, that a corresponding frequency domain bandwidth is 7 Hz to 23 Hz; or when determining that a value detected by the angular velocity sensor falls within a range from A1 to A2, the processor 614 may determine, based on Table 5, that a corresponding frequency domain bandwidth is 0.5 Hz to 40 Hz.

In some embodiments, when the watch 600 (such as the processor 614 in the watch 600) may select an appropriate frequency bandwidth based on the current status of the user, only a status (such as a movement status) of the watch 600 may be considered; only a current status (such as a skin status or a contact status with the first electrode and/or the second electrode) of the user, such as the body portion of the user in contact with the first electrode and/or the second electrode may be considered; or the status of the watch 600 and the current status of the user may be comprehensively considered, for example, not only the movement status of the watch 600, but also the current skin status of the user and the contact status of the body portion of the user with the first electrode and the second electrode are considered. For example, the watch 600 detects an impedance value by using the Bio-z sensor and detects an angular velocity value by using the angular velocity sensor, and when determining that the impedance value is greater than Z2 and the angular velocity value is greater than A2, the watch 600 (such as the processor 614 in the watch 600) determines that a frequency bandwidth is 7 Hz to 23 Hz. For another example, the watch detects an impedance value by using the Bio-z sensor and detects a pressure value by using the pressure sensor, and when determining that the impedance value is greater than Z2 and the pressure value is greater than P2, the watch 600 (such as the processor 614 in the watch 600) determines that a frequency bandwidth is 7 Hz to 23 Hz.

In some embodiments, after determining an appropriate frequency domain bandwidth based on a sensor parameter, the processor 614 may filter, based on the frequency domain bandwidth, electrical signals detected by the first electrode 616 and the second electrode 618. For example, the processor 614 determines that a frequency domain bandwidth is 7 Hz to 23 Hz, a frequency of an electrical signal detected by the first electrode 616 is 65 Hz to 150 Hz, and a frequency of an electrical signal detected by the second electrode 618 is 0.05 Hz to 120 Hz. The processor 614 may filter out an electrical signal outside 7 Hz to 23 Hz. In some examples, an electrical signal detected by the first electrode 616 may be (v1, t1), and an electrical signal detected by the second electrode 618 may be (v2, t2), where v1 and v2 represent voltages, t1 and t2 represent time, t1 is time at which the first electrode 616 collects the electrical signal v1, and t2 is time at which the second electrode 618 collects the electrical signal v2. Therefore, the processor 614 obtains a voltage change curve with time, and then can obtain a frequency domain change curve with time, that is, an ECG waveform.

In the foregoing embodiments, when the body portion of the user is in contact with the first electrode 616 and the second electrode 618, the first electrode 616 detects a first electrical signal, the second electrode 618 detects a second electrical signal, and the processor 614 selects an appropriate frequency bandwidth based on a current status of the user and then processes the first signal and the second signal based on the selected frequency bandwidth. In some other embodiments, the watch 600 stores an initial frequency bandwidth. For example, the initial frequency bandwidth may be a default frequency bandwidth, or a frequency bandwidth used when the watch 600 previously detects an ECG. After the first electrode 616 and the second electrode 618 detect electrical signals, the processor 614 may determine, based on a current status of the user, whether the initial frequency bandwidth needs to be adjusted; and if yes, the processor 614 adjusts the initial frequency bandwidth, and obtains an ECG signal by using an adjusted frequency domain bandwidth (for example, filters a first electrical signal and a second electrical signal by using the adjusted frequency bandwidth, and then obtains an ECG by using an electrical signal obtained after the filtering); or if no, the processor 614 obtains an ECG signal by using the initial frequency bandwidth.

For example, when the sensor subsystem 606 includes an angular velocity sensor, when determining that the angular velocity sensor detects a relatively large value, the processor 614 may reduce the initial frequency bandwidth; or when determining that the angular velocity sensor detects a relatively small value, the processor 614 may increase the initial frequency bandwidth. It should be noted that amplitude at which the initial frequency bandwidth is reduced/increased may be set by default, or may be customized by the user. This is not limited in this embodiment of this application.

For another example, when the sensor subsystem 606 includes a pressure sensor, when determining that the pressure sensor detects a relatively large pressure value, the processor 614 may reduce the initial frequency bandwidth; or when determining that the pressure sensor detects a relatively small pressure value, the processor 614 may increase the initial frequency bandwidth.

In some other embodiments, after the watch 600 enables the automatic ECG detection function, the body portion of the user is in contact with the first electrode and the second electrode on the watch 600. The first electrode detects a first electrical signal, and the second electrode detects a second electrical signal. The processor 614 may filter, by using initial frequency domain bandwidth, the electrical signals collected by the first electrode and the second electrode, and then generate an initial ECG signal based on an electrical signal obtained after the filtering. The processor 614 may determine, based on sensor data detected by the sensor subsystem 606, whether the initial ECG is accurate; if the initial ECG is inaccurate, adjust the initial frequency domain bandwidth, and then collect a new ECG based on an adjusted frequency bandwidth; and if the new ECG is inaccurate, adjust the frequency domain bandwidth again until a collected ECG is accurate. The following describes a possible implementation in which the processor 614 determines, based on sensor data, whether an ECG is accurate.

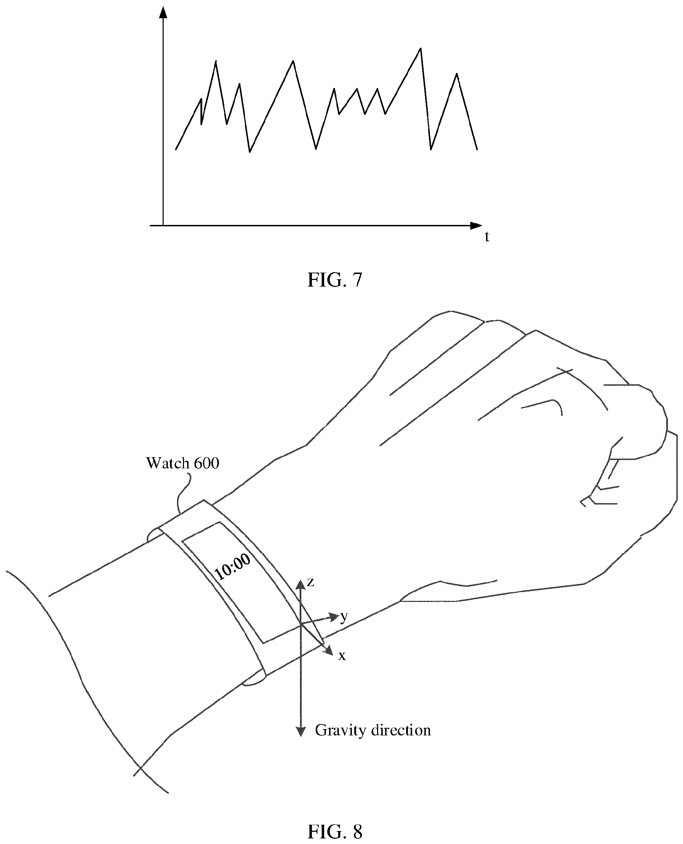

For example, FIG. 7 shows a schematic diagram of an initial ECG waveform obtained by the processor 614 by using an initial frequency bandwidth. The processor 614 determines a quantity of peaks or valleys included in unit duration (for example, 5 seconds, 10 seconds, or 20 seconds) in the initial ECG waveform, and denotes the quantity as N. When the sensor subsystem 606 includes a PPG sensor, the processor 164 may obtain a heart rate value of the human body by using the PPG sensor, and denote the value as M. The processor 614 determines whether a difference between M and N is greater than a threshold; and if yes, determines that an ECG is inaccurate; or if no, determines that an ECG is accurate. When determining that the ECG is inaccurate, the processor 164 may adjust the initial frequency domain bandwidth, and collect a new ECG, until the processor 163 determines that a difference between a heart rate value detected by the PPG sensor and a quantity that is of peaks or valleys in unit duration and that is obtained by using an ECG is less than or equal to the threshold.

In some other embodiments, a user performs various behavioral activities (such as running and lying still) in a process of wearing the watch 600. After a body portion of the user is in contact with the first electrode and the second electrode, the first electrode detects a first electrical signal, and the second electrode detects a second electrical signal. The watch 600 may determine, based on a current status of the user, whether to respond to the first electrical signal and the second electrical signal (for example, obtain an ECG based on the first electrical signal and the second electrical signal). The current status of the user may include a current movement speed, skin status, or the like of the user. In some examples, the processor 614 may determine, by using sensor data detected by the sensor subsystem 606, whether to respond to the first electrical signal and the second electrical signal (for example, obtain the ECG based on the first electrical signal and the second electrical signal).

For example, the sensor subsystem 606 may include a Bio-z sensor. Assuming that the processor determines that the Bio-z sensor detects a relatively small impedance value of a human body, for example, the impedance value is less than a threshold 1 (indicating that skin of the human body has a relatively large quantity of water stains, for example, sweats or is moist), the processor may not respond to the first electrical signal and the second electrical signal. In some embodiments, when the processor does not respond to the first signal and the second signal, the processor may output prompt information. For example, the prompt information may be "The body surface has many water stains and cannot be detected". In some embodiments, the prompt information may be information such as a text or an image on a display, voice information played by a speaker, or the like. Assuming that the processor determines that the Bio-z sensor detects an appropriate impedance value of the human body (for example, the impedance value falls within a range from the threshold 1 to a threshold 2), the processor may respond to the first electrical signal and the second electrical signal, for example, obtain a physiological parameter, such as an ECG signal, based on the first electrical signal and the second electrical signal.

For another example, the sensor subsystem 606 may include a motion sensor, such as an angular velocity sensor. In some embodiments, the watch 600 may establish a coordinate system (for example, a two-dimensional coordinate system or a three-dimensional coordinate system), for example, the watch 600 may establish the coordinate system by using a point (for example, a center point or an edge point of a display, or a point on a bezel of the watch) as an origin, and the angular velocity sensor may detect change speeds of angles between a gravity direction of the watch 600 and one or more of an x-axis, a y-axis, and a z-axis in the coordinate system. FIG. 8 is a schematic diagram in which a watch establishes a coordinate system according to an embodiment of this application. As shown in FIG. 8, the user wears the watch (for example, the watch 600). The watch 600 establishes a three-dimensional coordinate system at an edge point of the display, and the angular velocity sensor detects change speeds of angles between a gravity direction and one or more of an x-axis, a y-axis, and a z-axis in the coordinate system. When determining that the angular velocity sensor detects that the change speeds of the angles between the gravity direction and the one or more axes are not 0, the processor 614 determines that the watch 600 is in a moving state; or when determining that the angular velocity sensor detects that a change speed of an angle between the gravity direction and any axis is 0, the processor 614 determines that the watch 600 is in a still state.

In some embodiments, when determining, by using a value detected by the angular velocity sensor, that the watch 600 is in the still state, the processor 614 may respond to the first electrical signal and the second electrical signal (for example, obtain a physiological parameter based on the first electrical signal and the second electrical signal). In some other embodiments, when determining, by using a value detected by the angular velocity sensor, that the watch 600 is in the moving state, the processor 614 may not respond to the first electrical signal and the second electrical signal. In some embodiments, when the watch 600 does not respond to the first electrical signal and the second electrical signal, the watch 600 may output prompt information. For example, the prompt information may be "Detection cannot be performed during moving. Please stay still".

In some other embodiments, when determining that a value detected by the angular velocity sensor is greater than 0 and is less than a threshold, the processor 614 may respond to the first electrical signal and the second electrical signal, for example, obtain a physiological parameter, such as an ECG signal of the user, based on the first electrical signal and the second electrical signal; or when determining that a value detected by the angular velocity sensor is greater than the threshold, the processor 614 may not respond to the first electrical signal and the second electrical signal. That is, when detecting relatively small movement amplitude, the watch 600 may also obtain an ECG signal; or when detecting relatively large movement amplitude, the watch 600 may output prompt information, to give the user a prompt that the movement amplitude is quite large.

For another example, the sensor subsystem 606 may include a pressure sensor. When determining that a pressure value detected by the pressure sensor is less than a threshold, the processor 614 may respond to the first electrical signal and the second electrical signal, for example, generate a physiological parameter, such as an ECG, based on the first electrical signal and the second electrical signal; or when determining that a pressure value detected by the pressure sensor is greater than the threshold, the processor 614 may not respond to the first electrical signal and the second electrical signal. In some embodiments, the watch 600 may output prompt information. For example, the prompt information may be "Pressing strength is quite large. Please slightly touch".

In the foregoing embodiments, after the body portion of the user is in contact with the first electrode and the second electrode, the watch 600 selects an appropriate frequency bandwidth to obtain an ECG. That is, only after determining that the body portion of the user is in contact with the first electrode and the second electrode, the watch 600 performs processing such as bandwidth frequency selection, adjustment, or determining. In another embodiment of this application, after the watch 600 enables the automatic ECG detection function, the sensor subsystem 606 may be always in the enabled state. The sensor subsystem 606 may continuously detect sensor data in real time. The processor 614 may continuously select an appropriate frequency bandwidth in real time by using the sensor data. When the watch 600 determines that the body portion of the user is in contact with the first electrode and the second electrode (the first electrode detects the first electrical signal and the second electrode detects the second electrical signal), the processor 614 filters, by using a selected frequency bandwidth, the electrical signals collected by the first electrode and the second electrode, and then obtains an ECG signal based on an electrical signal obtained after the filtering. In this embodiment, the watch 600 continuously selects the appropriate frequency bandwidth in real time by using the sensor data detected by the sensor subsystem. Once the body portion of the user is in contact with the first electrode and the second electrode, the watch 600 performs processing by using the selected frequency bandwidth. This helps improve efficiency.

A process in which the watch 600 selects an appropriate frequency bandwidth based on sensor data is described above, and details are not described herein again.

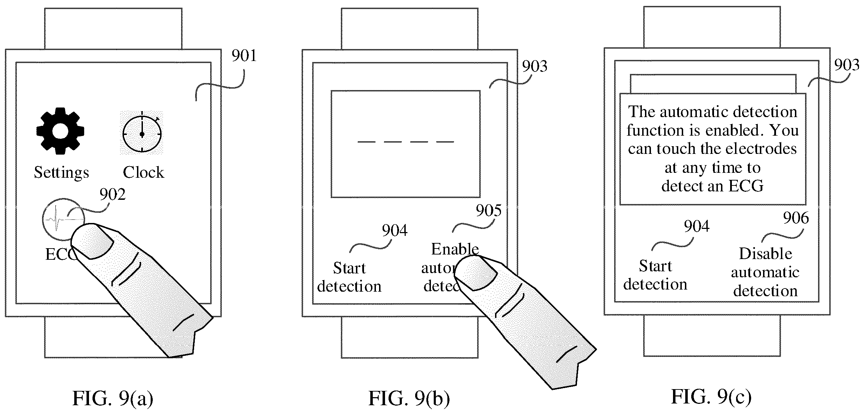

In some embodiments, the processor 614 may evaluate a health status of the user based on a detected ECG signal in a machine learning manner. Alternatively, the processor 614 may send, by using a wireless communications module or a mobile communications module, an ECG to a network side server or an electronic device, such as a mobile phone, connected to the wearable device 100 through Bluetooth, and the server or the mobile phone evaluates a health status of the user based on the ECG. For example, the wearable device 100 evaluates the health status of the user in the machine learning manner. The wearable device 100 may evaluate the health status of the user by using a stored model. The model may include a function relationship between an input parameter, a model parameter, and an output parameter. For example, after obtaining an ECG, the wearable device 100 may input the ECG to the model, that is, the ECG is used as an input parameter of the model; and run the model to obtain an output result, where the output result is health status information (for example, text information shown in FIG. 9) of the user. To improve accuracy of an output result of a model, the wearable device 100 may train the model. The model training may be as follows: An ECG and health status information corresponding to the ECG are input to the wearable device 100 (by using, for example, an input device or another electronic device, such as a network side server, connected to the wearable device 100); and the wearable device 100 inputs the ECG to the model as an input parameter, and runs the model to obtain an output result, where the output result is health status information. The wearable device 100 may compare the output result with the received health status information corresponding to the ECG. If a difference is relatively large, it indicates that accuracy of the model is relatively poor. Therefore, the wearable device 100 may adjust a model parameter, and run the model again, until a difference between an output result of the model and the health status information that corresponds to the ECG and that is received by the wearable device 100 is relatively small after a model parameter is adjusted. This process merely describes using one ECG as an input parameter of a model to train the model. In an actual application, several ECGs may be used as input parameters of a model (for example, several ECGs and health status information corresponding to each ECG are input to the wearable device 100), to obtain several output results. If differences between all the output results or most output results and received health statuses are relatively small, it indicates that accuracy of the model is relatively high. After the model training is completed, the wearable device 100 may use the model. For example, after obtaining the ECG based on the first electrical signal and the second electrical signal, the wearable device 100 inputs the ECG to the model, and runs the model to obtain an output result, that is, health status information.

It should be noted that in the conventional technology, when a user detects an ECG by using a wristband, there are a relatively large quantity of operation steps, such as lifting a wrist, waking up a screen of the wristband, tapping to enter a home screen, tapping an ECG APP in the home screen, being in contact with an electrode with a finger, starting measurement, and waiting for measurement to end. Therefore, the user can complete ECG detection only by performing a relatively large quantity of operation steps, causing very low ECG detection efficiency. The reason is as follows: In the conventional technology, an ECG detection function in the wristband needs to be enabled only after the ECG APP is entered. For example, after starting the ECG APP, the wristband may power on parts (such as the electrode) related to the ECG detection function, so that the parts are in an enabled state, and then the parts can execute the ECG detection function. When the wristband exits the ECG APP, the ECG detection function may be disabled. For example, the parts (such as the electrode) related to the ECG detection function are in a power-off or disabled state. Therefore, the user needs to manually enable the ECG detection function in the device each time the user detects an ECG by using the wristband. Operations are cumbersome, and response time is long, that is, relatively long time is needed to generate the ECG. In some embodiments of this application, for example, the wearable device 100 is the watch 600 shown in FIG. 6. The watch 600 may enable the automatic ECG detection function. When the watch 600 enables the automatic detection function, parts (such as the first electrode 616 and the second electrode 618) related to the ECG detection function are always in the enabled state. Therefore, provided that a body portion of a user is in contact with the electrodes on the watch 600, the electrodes can collect electrical signals of a human body in relatively short time, and then a physiological parameter, such as an ECG, of the human body is obtained. For example, after the watch 600 enables the automatic ECG detection function, when the display of the watch 600 currently displays an interface of another application (that is not an interface of an ECG APP) or displays a lock screen (an on-state lock screen or an off-state lock screen), provided that a body portion of a user is in contact with electrodes (for example, the first electrode 616 and the second electrode 618) on the wristband, the electrodes can collect electrical signals of a human body. Therefore, when a user needs to detect an ECG, the user can conveniently and quickly obtain the ECG without performing quite a lot of operation steps. This helps improve user experience.

For example, the wearable device 100 is the watch 600 shown in FIG. 6. The following describes several possible implementations in which the watch 600 enables the automatic ECG detection function.

In some embodiments, a control is displayed on the display of the watch 600, and when detecting an operation (such as an operation detected by using the crown 610 or the button 612) for the control, the watch 600 enables the automatic ECG detection function. FIG. 9(a) is a schematic diagram of a graphical user interface of a wearable device 100 according to an embodiment of this application. As shown in FIG. 9(a), the display of the watch 600 displays a home screen 901. The home screen 901 may include information such as time and weather, and further include icons of a plurality of applications, such as a "Clock" icon, a "Settings" icon, and an "ECG" icon 902. When detecting an operation for the icon 902 (for example, detecting the operation for the icon 902 by using the crown 610 or the button 612), the watch 600 displays an interface 903. As shown in FIG. 9(b), the interface 903 includes an electrocardiogram display area, a control 904, and a control 905. When the watch 600 detects an operation for the control 905, the watch 600 enables the automatic ECG detection function. When the watch 600 enables the automatic ECG detection function, the parts (such as the electrodes) related to the ECG detection function are always in the enabled state.

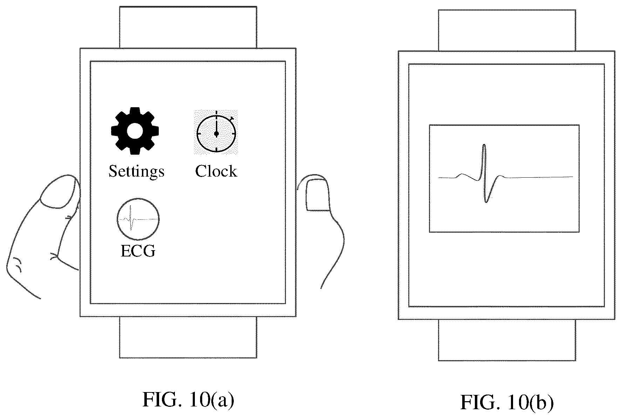

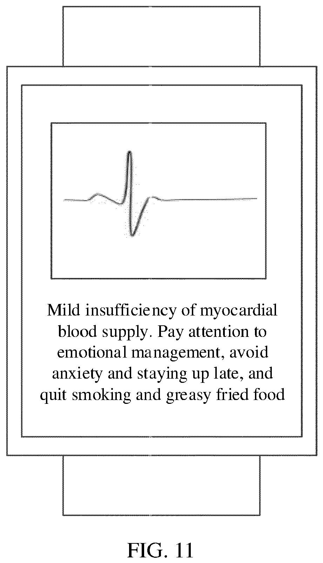

In some embodiments, after the watch 600 enables the automatic ECG detection function, regardless of any interface displayed on the display of the watch 600, the watch 600 displays an ECG provided that the electrodes detect electrical signals. For example, as shown in FIG. 10(a), the display of the watch 600 displays a home screen. After the electrodes of the watch 600 detect electrical signals, the display of the watch 600 displays an ECG, as shown in FIG. 10(b). In some other embodiments, the watch 600 may further display health status information of a user. For example, as shown in FIG. 11, the display of the watch 600 displays an ECG, and further displays health status information. In another embodiment, after the display of the watch 600 displays ECG preset time, ECG display may be automatically canceled. A specific value of the preset time is not limited in this embodiment of this application.

In some other embodiments, for example, the wearable device 100 is a wristband. The wristband may be communicatively connected to another electronic device, such as a mobile phone. After receiving an input operation used to enable the automatic ECG detection function, the another electronic device sends an instruction to the wristband. The instruction is used to instruct the wristband to enable the automatic ECG detection function. It should be understood that the another electronic device is, for example, a portable electronic device such as a smartphone, a tablet computer, a notebook computer, various wearable devices, a vehicle-mounted device, or a computer, or a non-portable electronic device such as a desktop computer. For example, for the mobile phone, various applications (application, app for short) such as a camera application, a messaging application, a multimedia messaging application, various mailbox applications, WeChat (WeChat), Tencent chat software (QQ), WhatsApp Messenger, Line (Line), instagram (instagram), Kakao Talk, and DingTalk may be installed in the mobile phone. In some embodiments, a specific application may be installed in the mobile phone. The specific application may be configured to control the wristband to be connected to the mobile phone, control the wristband to enable some functions, or the like. The specific application may be a dedicated application, or may be one or more of the foregoing applications. For example, a function used to control the wristband in the specific application is integrated into the one or more of the foregoing applications.

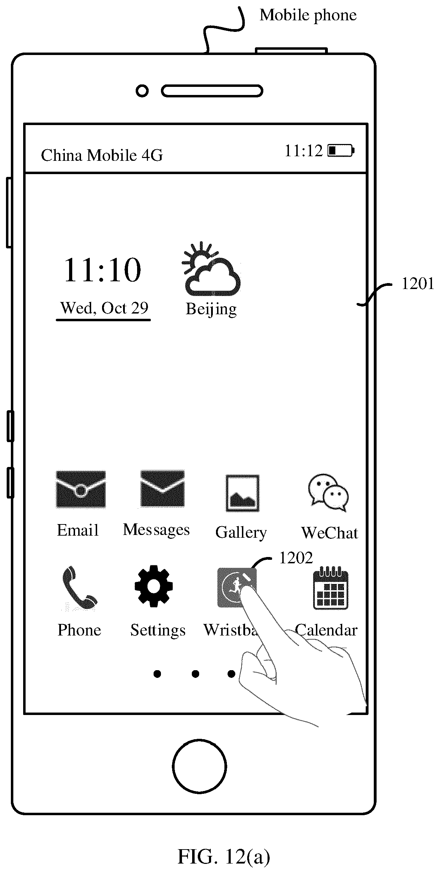

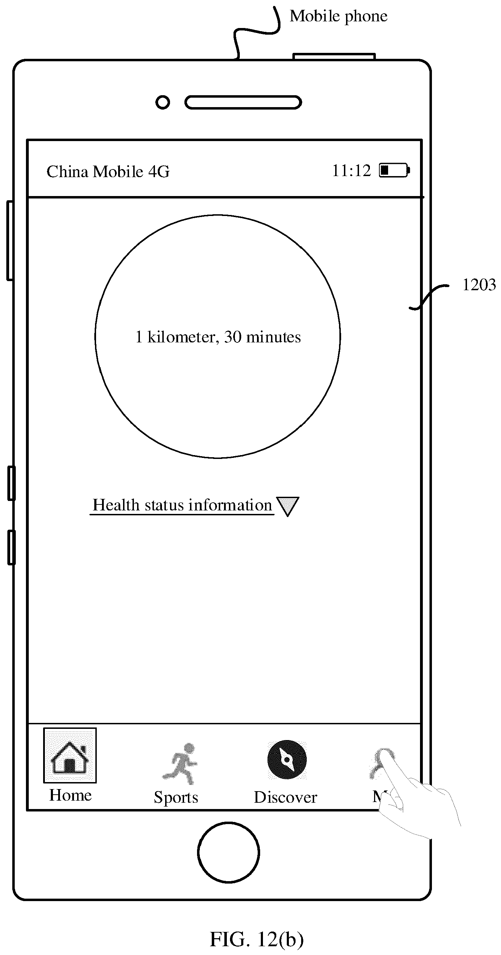

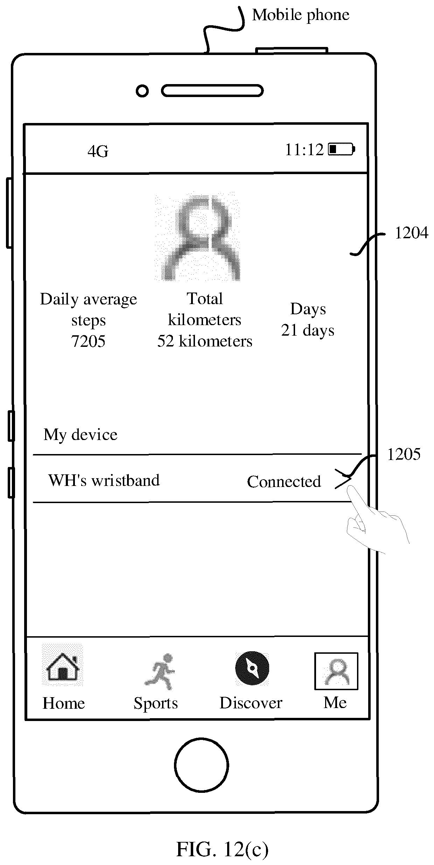

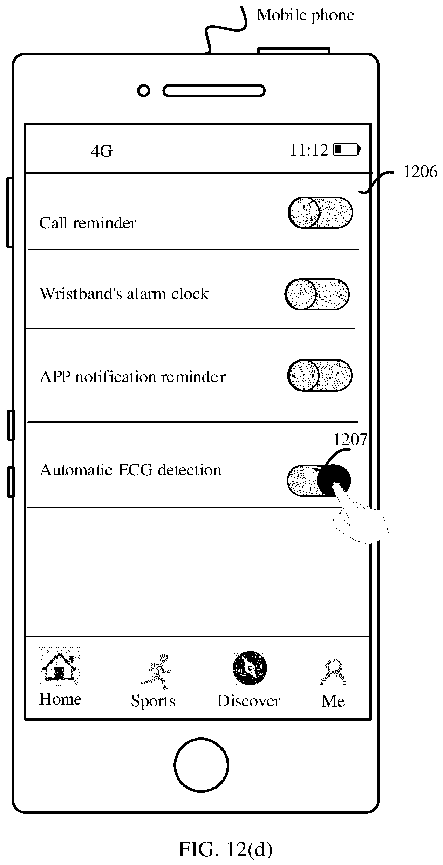

For example, FIG. 12(a) to FIG. 12(d) each are a schematic diagram of a graphical user interface of a mobile phone. As shown in FIG. 12(a), a display of the mobile phone displays a home screen (home screen) 1201. The home screen 1201 includes icons of a plurality of applications, including a "Wristband" icon 1202. When detecting an operation for the icon 1202, the mobile phone displays an interface 1203 shown in FIG. 12(b). The interface 1203 includes four controls: "Home", "Sports", "Discovery", and "My". When detecting an operation for the "My" control, the mobile phone displays an interface 1204 shown in FIG. 12(c). The interface 1204 displays a "My device" option, and the option includes identification information such as "WH's wristband" and "connected", and a control 1205. After detecting an operation for the control 1205, the mobile phone displays an interface 1206 shown in FIG. 12(d). The interface 1206 includes a plurality of options, including an "Automatic ECG detection" option and a control 1207. After detecting an operation used to activate the control 1207, the mobile phone sends an instruction to the wristband. The instruction is used to instruct to enable an automatic ECG detection function.

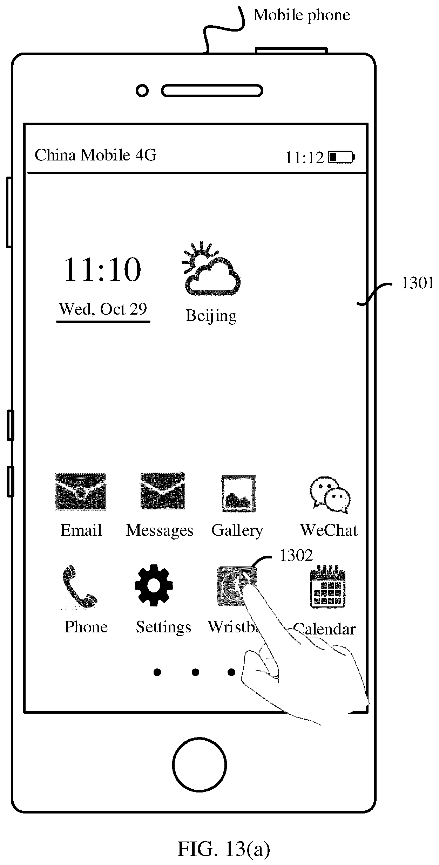

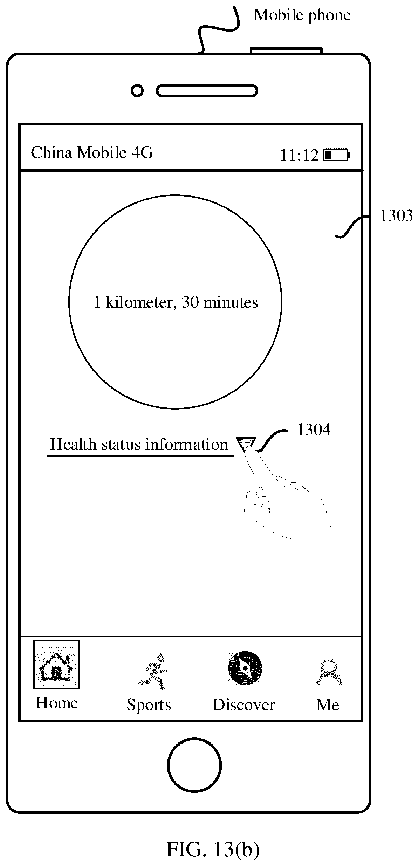

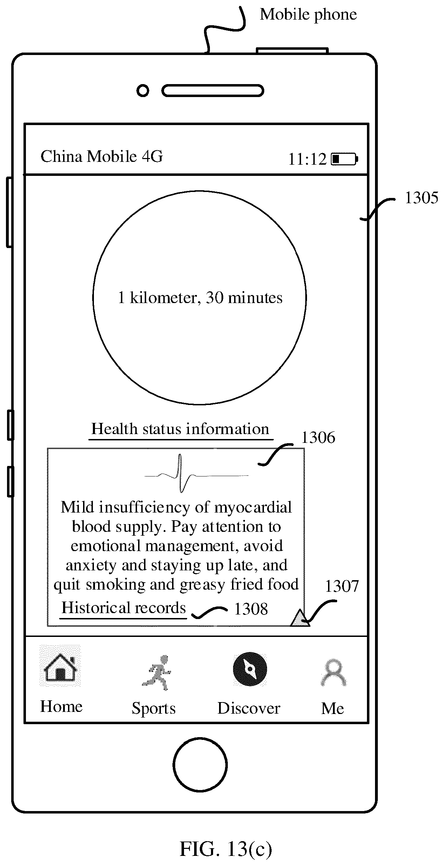

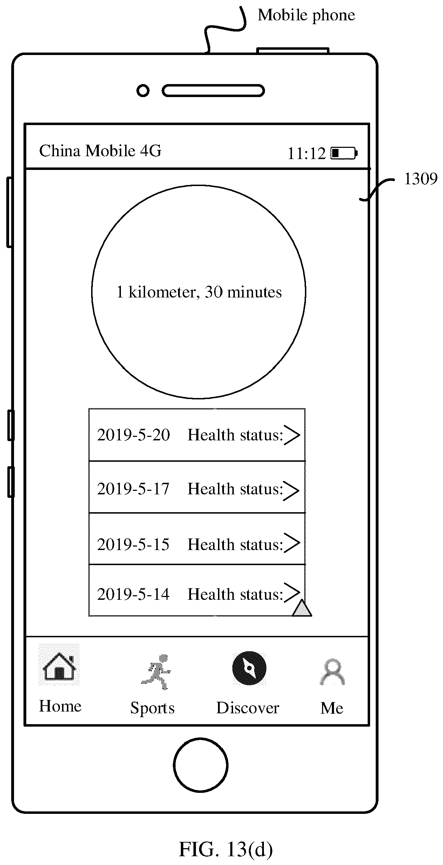

In some embodiments, after collecting an ECG of a user, the wristband may send the ECG to the mobile phone, or send health status information to the mobile phone. The mobile phone stores the ECG and the health status information. For example, FIG. 13(a) to FIG. 13(d) each are a schematic diagram of a graphical user interface of a mobile phone. As shown in FIG. 13(a), a display of the mobile phone displays a home screen (home screen) 1301. The home screen 1301 includes icons of a plurality of applications, including a "Wristband" icon 1302. When detecting an operation for the icon 1302, the mobile phone displays an interface 1303 shown in FIG. 13(b). The interface 1303 includes identification information "Health status information" and a button 1304. When detecting an operation for the button 1304, the mobile phone displays an interface 1305 shown in FIG. 13(c). The interface 1305 includes a window 1306 of an ECG and health status information. The window 1306 includes a button 1307 and a "Historical records" control 1308. When detecting an operation for the button 1307, the mobile phone closes the window 1306. After detecting an operation for the "Historical records" control 1308, the mobile phone displays an interface 1309 shown in FIG. 13(d). The interface 1309 includes a plurality of pieces of health status information, and a user can view detailed information of each piece of health status information.

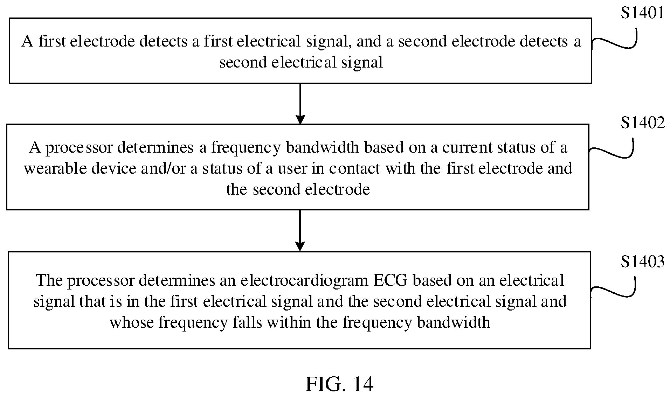

With reference to the foregoing embodiments and related accompanying drawings, an embodiment of this application provides an ECG detection method. The method may be implemented in the wearable device (such as a wristband or a watch) shown in any one of FIG. 1A to FIG. 6. The wearable device may include a processor, a first electrode, and a second electrode. For the processor, the first electrode, and the second electrode, refer to the foregoing descriptions. As shown in FIG. 14, the method may include the following steps.

1401: The first electrode detects a first electrical signal, and the second electrode detects a second electrical signal. In some embodiments, for example, in the watch 600 shown in FIG. 6, the first electrode (for example, the electrode 616) may be disposed on the lower surface 605, and the second electrode (for example, the electrode 618) may be disposed on the button 612. Assuming that a left wrist of a user wears the watch 600, the left wrist of the user may be in contact with the first electrode on the lower surface 605 of the watch 600, and a right hand finger of the user may be in contact with the second electrode on the button 612. In this way, the first electrode detects the first electrical signal, and the second electrode detects the second electrical signal.

1402: The processor determines a frequency bandwidth based on a current status of the wearable device and/or a status of the user in contact with the first electrode and the second electrode.

In some embodiments, the current status of the wearable device may include a movement status of the wearable device, or the like, and the status of the user in contact with the first electrode and the second electrode may include a skin status of a body portion of the user in contact with the first electrode and the second electrode, and/or a contact status of the user with the first electrode and/or the second electrode. In some embodiments, the wearable device may detect the movement status of the wearable device, the skin status of the body portion of the user in contact with the first electrode and the second electrode, the contact status of the user with the first electrode and/or the second electrode, or the like by using a sensor. For example, the wearable device may include a motion sensor (such as an angular velocity sensor), to detect the movement status of the wearable device. In some other embodiments, the wearable device may include a bioimpedance zinvasion sensor, a pressure sensor, a capacitive sensor, or the like. The bioimpedance zinvasion sensor may be configured to detect the skin status of the user (for example, the bioimpedance zinvasion sensor is disposed on the first electrode and/or the second electrode, and may be configured to detect the skin status of the body portion of the user in contact with the first electrode and/or the second electrode). The pressure sensor and/or the capacitive sensor may be configured to detect the contact status of the user with the first electrode and/or the second electrode.

In some embodiments, the wearable device may select an appropriate frequency bandwidth based on the status of the wearable device and/or the status of the user in contact with the first electrode and the second electrode, and then obtain an ECG based on the frequency bandwidth. Therefore, regardless of a movement status, such as a running state or a still state, of the user, the user can detect a relatively accurate ECG by using the wearable device, for ease of use by the user.

In some embodiments, an execution sequence of 1401 and 1402 is not limited. For example, a case in which 1401 is performed before 1402 is as follows: Only after the user is in contact with the first electrode and the second electrode, the wearable device selects the appropriate frequency bandwidth based on the status of the wearable device and/or the status of the user (for example, sensor data obtained by using the sensor), processes (for example, filter), based on the frequency bandwidth, the electrical signals detected by the first electrode and the second electrode, and then obtains the ECG based on an electrical signal obtained after the processing. For another example, a case in which 1402 is performed before 1401 is as follows: The sensor (for example, the motion sensor, the bioimpedance zinvasion sensor, the pressure sensor, and/or the capacitive sensor) in the wearable device is in an enabled state, and the wearable device determines an appropriate frequency bandwidth in real time by using sensor data detected by the sensor. After the user is in contact with the first electrode and the second electrode and the first electrode and the second electrode detect the electrical signals, the wearable device may process (for example, filter), based on the determined frequency bandwidth, the electrical signals detected by the first electrode and the second electrode, and then obtain an ECG based on an electrical signal obtained after the processing.

1403: The processor determines the electrocardiogram ECG based on an electrical signal that is in the first electrical signal and the second electrical signal and whose frequency falls within the frequency bandwidth.

In some embodiments, the wearable device may include a filtering apparatus. The filtering apparatus may filter the electrical signals detected by the first electrode and the second electrode. For example, after collecting the electrical signals, the first electrode and the second electrode may provide the electrical signals for the filtering apparatus. The filtering apparatus may filter, by using a specific frequency bandwidth, the electrical signals provided by the first electrode and the second electrode. For example, frequencies of the electrical signals detected by the first electrode and the second electrode fall within a range of 0.05 Hz to 150 Hz. The filtering apparatus is configured to filter, by using a frequency bandwidth of 7 Hz to 23 Hz, the electrical signals of a human body that are collected by an electrode group, a frequency of an electrical signal retained after the filtering falls within the range of 7 Hz to 23 Hz, and an electrical signal outside the frequency range of 7 Hz to 23 Hz is filtered out. The filtering apparatus provides the retained electrical signal for the processor 103, and the processor 103 obtains a physiological parameter, such as an ECG signal, of the human body, based on the electrical signal.

Usually, an electrical signal generated by a human body has a specific frequency, and a bandwidth of the frequency ranges from 0.05 Hz to 150 Hz. When the human body is in a moving state, respiration of the human body, an action of the body, and the like all affect generation of an electrical signal. Therefore, electrical signals (whose frequencies range from 0.05 Hz to 150 Hz) generated by the human body include a relatively large amount of noise. If a device configured to detect an ECG generates an ECG based on electrical signals that include noise, accuracy of the obtained ECG is affected. Therefore, in this embodiment of this application, the wearable device may select the appropriate frequency bandwidth based on the status of the wearable device and/or the status of the user in contact with the first electrode and the second electrode, and then obtain the ECG based on the frequency bandwidth. Therefore, regardless of a movement status, such as a running state or a still state, of the user, the user can detect a relatively accurate ECG by using the wearable device, for ease of use by the user.

The implementations of this application may be randomly combined to achieve different technical effects.

In the embodiments provided in this application, the method provided in the embodiments of this application is described from a perspective of the wearable device (for example, the watch 600) used as an execution body. To implement the functions in the methods provided in the embodiments of this application, the electronic device may include a hardware structure and/or a software module, and implement the functions in a form of the hardware structure, the software module, or a combination of the hardware structure and the software module. Whether a function in the foregoing functions is performed by using the hardware structure, the software module, or the combination of the hardware structure and the software module depends on particular applications and design constraints of the technical solutions.

According to the context, the term "when" or "after" used in the foregoing embodiments may be interpreted as a meaning of "if", "after", "in response to determining", or "in response to detecting". Similarly, according to the context, the phrase "when it is determined that" or "if (a stated condition or event) is detected" may be interpreted as a meaning of "when it is determined that", "in response to determining", "when (a stated condition or event) is detected", or "in response to detecting (a stated condition or event)". In addition, in the foregoing embodiments, relationship terms such as first and second are used to distinguish one entity from another entity, but do not limit any actual relationship and sequence between these entities.

All or some of the foregoing embodiments may be implemented by using software, hardware, firmware, or any combination thereof. When software is used to implement the embodiments, the embodiments may be implemented completely or partially in a form of a computer program product. The computer program product includes one or more computer instructions. When the computer program instructions are loaded and executed on a computer, the procedures or the functions according to the embodiments of the present invention are all or partially generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by a computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive (Solid-State Drive, SSD)), or the like.

It should be noted that a part of this patent application document includes content protected by the copyright. The copyright owner reserves the copyright except copies made for the patent documents or the recorded content of the patent documents in the China National Intellectual Property Administration.

## Claims

1. An ECG detection method, applied to a wearable device (100), wherein the wearable device (100) comprises a processor (103), a first electrode (105A), and a second electrode (105B), and the method comprises:
detecting (S1401), by the first electrode (105A), a first electrical signal, and detecting, by the second electrode (105B), a second electrical signal;
determining (S1402), by the processor (103), a frequency bandwidth based on a current status of the wearable device (100) and/or a status of a user in contact with the first electrode (105A) and the second electrode (105B), the first electrode (105A) and the second electrode (105B) being disposed on one or more outer surfaces of the wearable device; and
determining (S1403), by the processor (103), an electrocardiogram, ECG, based on an electrical signal that is in the first electrical signal and the second electrical signal and whose frequency falls within the frequency bandwidth; and
wherein the determining (S1402), by the processor (103), a frequency bandwidth based on a current status of the wearable device (100) comprises:
determining, by the processor (103), a current movement status of the wearable device (100) based on a motion sensor in the wearable device (100); and
determining, by the processor (103), the frequency bandwidth based on the movement status.

2. The method according to claim 1, wherein the determining (S1402), by the processor (103), a frequency bandwidth based on a status of a user in contact with the first electrode (105A) and the second electrode (105B) comprises:
determining, by the processor (103) based on an impedance value detected by a bioimpedance sensor, Bio-z sensor in the wearable device (100), a skin status of a body portion of the user in contact with the first electrode (105A) and/or the second electrode (105B); and
determining, by the processor (103), the frequency bandwidth based on the skin status of the user.

3. The method according to any one of claims 1 to 2, wherein the determining (S1402), by the processor (103), a frequency bandwidth based on a status of a user in contact with the first electrode (105A) and the second electrode (105B) comprises:
determining, by the processor (103), a contact status of the user with the first electrode (105A) and the second electrode (105B) based on a pressure sensor (106B) and/or a capacitive sensor (106D) in the wearable device (100); and
determining, by the processor (103), the frequency bandwidth based on the contact status.

4. The method according to any one of claims 1 to 3, wherein before the detecting (S1401), by the first electrode (105A), a first electrical signal, and detecting, by the second electrode (105B), a second electrical signal, the method further comprises:
displaying, by a display of the wearable device (100), an off-state lock screen, an on-state lock screen, or a home screen.

5. The method according to any one of claims 1 to 4, wherein before the detecting (S1401), by the first electrode (105A), a first electrical signal, and detecting, by the second electrode (105B), a second electrical signal, the method further comprises:
detecting an input operation (101); and
enabling an automatic ECG detection function in response to the input operation, wherein
the first electrode (105A) and the second electrode (105B) are always in an enabled state after the wearable device (100) enables the automatic ECG detection function.

6. The method according to any one of claims 1 to 5, wherein the method further comprises:
displaying, by the wearable device (100), a waveform corresponding to the ECG and health status information corresponding to the ECG.

7. A wearable device (100), wherein the wearable device (100) comprises a processor (103), a first electrode (105A), and a second electrode (105B);
the first electrode (105A) is configured to detect (S1401) a first electrical signal;
the second electrode (105B) is configured to detect (S1401) a second electrical signal;
the processor (103) is configured to determine (S1402) a frequency bandwidth based on a current status of the wearable device (100) and/or a status of a user in contact with the first electrode (105A) and the second electrode (105B), the first electrode (105A) and the second electrode (105B) being disposed on one or more outer surfaces of the wearable device; and
the processor (103) is further configured to determine (S1403) an electrocardiogram ECG based on an electrical signal that is in the first electrical signal and the second electrical signal and whose frequency falls within the frequency bandwidth; and
wherein the wearable device (100) comprises a motion sensor (106F);
the motion sensor (106F)is configured to detect a movement status of the wearable device (100); and
the processor (103) is specifically configured to determine (S1402) the frequency bandwidth based on the movement status.

8. The wearable device (100) according to claim 7, wherein the wearable device (100) comprises a bioimpedance sensor (106C), Bio-z sensor, and the Bio-z sensor (106C) is disposed on the first electrode (105A) and/or the second electrode (105B);
the Bio-z sensor (106C) is configured to detect a skin status of a body portion of the user in contact with the first electrode (105A) and/or the second electrode (105B); and
the processor (103) is specifically configured to determine (S1402) the frequency bandwidth based on the skin status of the user.

9. The wearable device (100) according to any one of claims 7 to 8, wherein the wearable device (100) comprises a pressure sensor (106B) and/or a capacitive sensor (106D); and the pressure sensor (106B) is disposed on the first electrode (105A) and/or the second electrode (105B), and/or the capacitive sensor (106D) is disposed on the first electrode (105A) and/or the second electrode (105B);
the pressure sensor (106B) and/or the capacitive sensor (106D) are/is configured to detect a contact status of the user with the first electrode (105A) and/or the second electrode (105B); and
the processor (103) is specifically configured to determine (S1402) the frequency bandwidth based on the contact status.

10. The wearable device (100) according to any one of claims 7 to 9, wherein the wearable device (100) further comprises a display; and
before the first electrode (105A) detects (S1401) the first electrical signal, and the second electrode (105B) detects (S1401) the second electrical signal, the display displays an off-state lock screen, an on-state lock screen, or a home screen.

11. The wearable device (100) according to any one of claims 7 to 10, wherein the wearable device (100) further comprises an input device (101), and the input device (101) is configured to detect an input operation; and
the processor (103) is further configured to enable an automatic ECG detection function in response to the input operation, so that the first electrode (105A) and the second electrode (105B) are always in an enabled state.

12. The wearable device (100) according to any one of claims 7 to 11, wherein the wearable device (100) comprises the display, and the display is configured to display a waveform corresponding to the ECG and health status information corresponding to the ECG.

13. A program product, wherein the program product comprises instructions, and when the instructions are run on a computer, the computer is enabled to perform the method according to any one of claims 1 to 6, wherein the computer instructions are executed on the specific wearable device according to one or more of claims 7 to 12.

## Patentansprüche

1. Verfahren zur EKG-Detektion, angewandt auf eine am Körper tragbare Vorrichtung (100), wobei die am Körper tragbare Vorrichtung (100) einen Prozessor (103), eine erste Elektrode (105A) und eine zweite Elektrode (105B) umfasst und wobei das Verfahren Folgendes umfasst:
Detektieren (S1401), durch die erste Elektrode (105A), eines ersten elektrischen Signals, und Detektieren, durch die zweite Elektrode (105B), eines zweiten elektrischen Signals;
Bestimmen (S1402), durch den Prozessor (103), einer Frequenzbandbreite basierend auf einem aktuellen Status der am Körper tragbaren Vorrichtung (100) und/oder einem Status eines Benutzers in Kontakt mit der ersten Elektrode (105A) und der zweiten Elektrode (105B), wobei die erste Elektrode (105A) und die zweite Elektrode (105B) auf einer oder mehreren äußeren Oberflächen der am Körper tragbaren Vorrichtung angeordnet sind; und
Bestimmen (S1403), durch den Prozessor (103), eines Elektrokardiogramms, EKG, basierend auf einem elektrischen Signal, das in dem ersten elektrischen Signal und dem zweiten elektrischen Signal ist und dessen Frequenz in die Frequenzbandbreite fällt; und
wobei das Bestimmen (S1402), durch den Prozessor (103), einer Frequenzbandbreite basierend auf einem aktuellen Status der am Körper tragbaren Vorrichtung (100) Folgendes umfasst:
Bestimmen, durch den Prozessor (103), eines aktuellen Bewegungsstatus der am Körper tragbaren Vorrichtung (100) basierend auf einem Bewegungssensor in der am Körper tragbaren Vorrichtung (100); und
Bestimmen, durch den Prozessor (103), der Frequenzbandbreite basierend auf dem Bewegungsstatus.

2. Verfahren nach Anspruch 1, wobei das Bestimmen (S1402), durch den Prozessor (103), einer Frequenzbandbreite basierend auf einem Status eines Benutzers in Kontakt mit der ersten Elektrode (105A) und der zweiten Elektrode (105B) Folgendes umfasst:
Bestimmen, durch den Prozessor (103) basierend auf einem Impedanzwert, detektiert durch einen Bioimpedanzsensor, Bio-z-Sensor, in der am Körper tragbaren Vorrichtung (100), eines Hautstatus eines Körperteils des Benutzers in Kontakt mit der ersten Elektrode (105A) und/oder der zweiten Elektrode (105B); und
Bestimmen, durch den Prozessor (103), der Frequenzbandbreite basierend auf dem Hautstatus des Benutzers.

3. Verfahren nach einem der Ansprüche 1 bis 2, wobei das Bestimmen (S1402), durch den Prozessor (103), einer Frequenzbandbreite basierend auf einem Status eines Benutzers in Kontakt mit der ersten Elektrode (105A) und der zweiten Elektrode (105B) Folgendes umfasst:
Bestimmen, durch den Prozessor (103), eines Kontaktstatus des Benutzers mit der ersten Elektrode (105A) und der zweiten Elektrode (105B) basierend auf einem Drucksensor (106B) und/oder einem kapazitiven Sensor (106D) in der am Körper tragbaren Vorrichtung (100); und
Bestimmen, durch den Prozessor (103), der Frequenzbandbreite basierend auf dem Kontaktstatus.

4. Verfahren nach einem der Ansprüche 1 bis 3, wobei vor dem Detektieren (S1401), durch die erste Elektrode (105A), eines ersten elektrischen Signals und Detektieren, durch die zweite Elektrode (105B), eines zweiten elektrischen Signals das Verfahren ferner Folgendes umfasst:
Anzeigen, durch eine Anzeige der am Körper tragbaren Vorrichtung (100), eines Aus-Zustand-Sperrbildschirms, eines Ein-Zustand-Sperrbildschirms oder eines Startbildschirms.

5. Verfahren nach einem der Ansprüche 1 bis 4, wobei vor dem Detektieren (S1401), durch die erste Elektrode (105A), eines ersten elektrischen Signals und Detektieren, durch die zweite Elektrode (105B), eines zweiten elektrischen Signals das Verfahren ferner Folgendes umfasst:
Detektieren einer Eingabeoperation (101); und
Aktivieren einer automatischen EKG-Detektionsfunktion in Reaktion auf die Eingabeoperation, wobei
die erste Elektrode (105A) und die zweite Elektrode (105B) immer in einem aktivierten Zustand sind, nachdem die am Körper tragbare Vorrichtung (100) die automatische EKG-Detektionsfunktion aktiviert.

6. Verfahren nach einem der Ansprüche 1 bis 5, wobei das Verfahren ferner Folgendes umfasst:
Anzeigen, durch die am Körper tragbare Vorrichtung (100), einer Wellenform entsprechend dem EKG und von Gesundheitsstatusinformationen entsprechend dem EKG.

7. Am Körper tragbare Vorrichtung (100), wobei die am Körper tragbare Vorrichtung (100) einen Prozessor (103), eine erste Elektrode (105A) und eine zweite Elektrode (105B) umfasst;
die erste Elektrode (105A) ausgelegt ist zum Detektieren (S1401) eines ersten elektrischen Signals;
die zweite Elektrode (105B) ausgelegt ist zum Detektieren (S1401) eines zweiten elektrischen Signals;
der Prozessor (103) ausgelegt ist zum Bestimmen (S1402) einer Frequenzbandbreite basierend auf einem aktuellen Status der am Körper tragbaren Vorrichtung (100) und/oder einem Status eines Benutzers in Kontakt mit der ersten Elektrode (105A) und der zweiten Elektrode (105B), wobei die erste Elektrode (105A) und die zweite Elektrode (105B) auf einer oder mehreren äußeren Oberflächen der am Körper tragbaren Vorrichtung angeordnet sind; und
der Prozessor (103) ferner ausgelegt ist zum Bestimmen (S1403) eines Elektrokardiogramms, EKG, basierend auf einem elektrischen Signal, das in dem ersten elektrischen Signal und dem zweiten elektrischen Signal ist und dessen Frequenz in die Frequenzbandbreite fällt; und
wobei die am Körper tragbare Vorrichtung (100) einen Bewegungssensor (106F) umfasst;
der Bewegungssensor (106F) ausgelegt ist zum Detektieren eines Bewegungsstatus der am Körper tragbaren Vorrichtung (100); und
der Prozessor (103) speziell ausgelegt ist zum Bestimmen (S1402) der Frequenzbandbreite basierend auf dem Bewegungsstatus.

8. Am Körper tragbare Vorrichtung (100) nach Anspruch 7, wobei die am Körper tragbare Vorrichtung (100) einen Bioimpedanzsensor (106C), Bio-z-Sensor, umfasst und der Bio-z-Sensor (106C) an der ersten Elektrode (105A) und/oder der zweiten Elektrode (105B) angeordnet ist;
der Bio-z-Sensor (106C) ausgelegt ist zum Detektieren eines Hautstatus eines Körperteils des Benutzers in Kontakt mit der ersten Elektrode (105A) und/oder der zweiten Elektrode (105B); und
der Prozessor (103) speziell ausgelegt ist zum Bestimmen (S1402) der Frequenzbandbreite basierend auf dem Hautstatus des Benutzers.

9. Am Körper tragbare Vorrichtung (100) nach einem der Ansprüche 7 bis 8, wobei die am Körper tragbare Vorrichtung (100) einen Drucksensor (106B) und/oder einen kapazitiven Sensor (106D) umfasst; und wobei der Drucksensor (106B) an der ersten Elektrode (105A) und/oder der zweiten Elektrode (105B) angeordnet ist und/oder der kapazitive Sensor (106D) an der ersten Elektrode (105A) und/oder der zweiten Elektrode (105B) angeordnet ist;
der Drucksensor (106B) und/oder der kapazitive Sensor (106D) ausgelegt sind/ist zum Detektieren eines Kontaktstatus des Benutzers mit der ersten Elektrode (105A) und/oder der zweiten Elektrode (105B); und
der Prozessor (103) speziell ausgelegt ist zum Bestimmen (S1402) der Frequenzbandbreite basierend auf dem Kontaktstatus.

10. Am Körper tragbare Vorrichtung (100) nach einem der Ansprüche 7 bis 9, wobei die am Körper tragbare Vorrichtung (100) ferner eine Anzeige umfasst; und
wobei, bevor die erste Elektrode (10SA) das erste elektrische Signal detektiert (S1401) und die zweite Elektrode (105B) das zweite elektrische Signal detektiert (S1401), die Anzeige einen Aus-Zustand-Sperrbildschirm, einen Ein-Zustand-Sperrbildschirm oder einen Startbildschirm anzeigt.

11. Am Körper tragbare Vorrichtung (100) nach einem der Ansprüche 7 bis 10, wobei die am Körper tragbare Vorrichtung (100) ferner eine Eingabevorrichtung (101) umfasst und die Eingabevorrichtung (101) ausgelegt ist zum Detektieren einer Eingabeoperation; und
der Prozessor (103) ferner ausgelegt ist zum Aktivieren einer automatischen EKG-Detektionsfunktion in Reaktion auf die Eingabeoperation, sodass die erste Elektrode (105A) und die zweite Elektrode (105B) immer in einem aktivierten Zustand sind.

12. Am Körper tragbare Vorrichtung (100) nach einem der Ansprüche 7 bis 11, wobei die am Körper tragbare Vorrichtung (100) die Anzeige umfasst und die Anzeige ausgelegt ist zum Anzeigen einer Wellenform entsprechend dem EKG und von Gesundheitsstatusinformationen entsprechend dem EKG.

13. Programmprodukt, wobei das Programmprodukt Anweisungen umfasst, und wobei, wenn die Anweisungen auf einem Computer ausgeführt werden, der Computer in die Lage versetzt wird, das Verfahren nach einem der Ansprüche 1 bis 6 durchzuführen, wobei die Computeranweisungen auf der spezifischen am Körper tragbaren Vorrichtung nach einem oder mehreren der Ansprüche 7 bis 12 ausgeführt werden.

## Revendications

1. Procédé de détection ECG, appliqué à un dispositif portable (100), dans lequel le dispositif portable (100) comprend un processeur (103), une première électrode (105A), et une seconde électrode (105B), et le procédé comprend :
la détection (S1401), par la première électrode (105A), d'un premier signal électrique, et la détection, par la seconde électrode (105B), d'un second signal électrique ;
la détermination (S1402), par le processeur (103), d'une largeur de bande de fréquences sur la base d'un état actuel du dispositif portable (100) et/ou d'un état d'un utilisateur en contact avec la première électrode (105A) et la seconde électrode (105B), la première électrode (105A) et la seconde électrode (105B) étant disposées sur une ou plusieurs surfaces externes du dispositif portable ; et
la détermination (S1403), par le processeur, d'un électrocardiogramme, ECG, sur la base d'un signal électrique qui est dans le premier signal électrique et le second signal électrique et dont la fréquence se trouve dans la largeur de bande de fréquences ; et
dans lequel la détermination (S1402), par le processeur (103), d'une largeur de bande de fréquences sur la base d'un état actuel du dispositif portable (100) comprend :
la détermination, par le processeur (103), d'un état de mouvement actuel du dispositif portable (100) sur la base d'un capteur de mouvement dans le dispositif portable (100) ; et
la détermination, par le processeur (103), de la largeur de bande de fréquences sur la base de l'état de mouvement.

2. Procédé selon la revendication 1, dans lequel la détermination (S1402), par le processeur (103), d'une largeur de bande de fréquences sur la base d'un état d'un utilisateur en contact avec la première électrode (105A) et la seconde électrode (105B) comprend :
la détermination, par le processeur (103) sur la base d'une valeur d'impédance détectée par un capteur de bioimpédance, capteur Bio-z, dans le dispositif portable (100), d'un état de peau d'une partie de corps de l'utilisateur en contact avec la première électrode (105A) et/ou la seconde électrode (105B) ; et
la détermination, par le processeur (103), de la largeur de bande de fréquences sur la base de l'état de peau de l'utilisateur.

3. Procédé selon l'une quelconque des revendications 1 à 2, dans lequel la détermination (S1402), par le processeur (103), d'une largeur de bande de fréquences sur la base d'un état d'un utilisateur en contact avec la première électrode (105A) et la seconde électrode (105B) comprend :
la détermination, par le processeur (103), d'un état de contact de l'utilisateur avec la première électrode (105A) et la seconde électrode (105B) sur la base d'un capteur de pression (106B) et/ou d'un capteur capacitif (106D) dans le dispositif portable (100) ; et
la détermination, par le processeur (103), de la largeur de bande de fréquences sur la base de l'état de contact.

4. Procédé selon l'une quelconque des revendications 1 à 3, dans lequel avant la détection (S1401), par la première électrode (105A), d'un premier signal électrique, et la détection, par la seconde électrode (105B), d'un second signal électrique, le procédé comprend en outre :
l'affichage, par un dispositif d'affichage du dispositif portable (100), d'un écran de verrouillage à l'état éteint, d'un écran de verrouillage à l'état allumé, ou d'un écran d'accueil.

5. Procédé selon l'une quelconque des revendications 1 à 4, dans lequel avant la détection (S1401), par la première électrode (105A), d'un premier signal électrique, et la détection, par la seconde électrode (105B), d'un second signal électrique, le procédé comprend en outre :
la détection d'une opération d'entrée (101) ; et
l'activation d'une fonction de détection ECG automatique en réponse à l'opération d'entrée, dans lequel
la première électrode (105A) et la seconde électrode (105B) sont toujours dans un état activé après que le dispositif portable (100) active la fonction de détection ECG automatique.

6. Procédé selon l'une quelconque des revendications 1 à 5, dans lequel le procédé comprend en outre :
l'affichage, par le dispositif portable (100), d'une forme d'onde correspondant à l'ECG et d'informations d'état de santé correspondant à l'ECG.

7. Dispositif portable (100), dans lequel le dispositif portable (100) comprend un processeur (103), une première électrode (105A), et une seconde électrode (105B) ; la première électrode (105A) est configurée pour détecter (S1401) un premier signal électrique ;
la seconde électrode (105B) est configurée pour détecter (S1401) un second signal électrique ;
le processeur (103) est configuré pour déterminer (S1402) une largeur de bande de fréquences sur la base d'un état actuel du dispositif portable (100) et/ou d'un état d'un utilisateur en contact avec la première électrode (105A) et la seconde électrode (105B), la première électrode (105A) et la seconde électrode (105B) étant disposées sur une ou plusieurs surfaces externes du dispositif portable ; et
le processeur (103) est en outre configuré pour déterminer (S1403) un électrocardiogramme, ECG, sur la base d'un signal électrique qui est dans le premier signal électrique et le second signal électrique et dont la fréquence se trouve dans la largeur de bande de fréquences ; et
dans lequel le dispositif portable (100) comprend un capteur de mouvement (106F) ; le capteur de mouvement (106F) est configuré pour détecter un état de mouvement du dispositif portable (100) ; et
le processeur (103) est spécifiquement configuré pour déterminer (S1402) la largeur de bande de fréquences sur la base de l'état de mouvement.

8. Dispositif portable (100) selon la revendication 7, dans lequel le dispositif portable (100) comprend un capteur de bioimpédance (106C), capteur Bio-Z, et le capteur Bio-z (106C) est disposé sur la première électrode (105A) et/ou la seconde électrode (105B) ;
le capteur Bio-z (106C) est configuré pour détecter un état de peau d'une partie de corps de l'utilisateur en contact avec la première électrode (105A) et/ou la seconde électrode (105B) ; et
le processeur (103) est spécifiquement configuré pour déterminer (S1402) la largeur de bande de fréquences sur la base de l'état de peau de l'utilisateur.

9. Dispositif portable (100) selon l'une quelconque des revendications 7 à 8, dans lequel le dispositif portable (100) comprend un capteur de pression (106B) et/ou un capteur capacitif (106D) ; et le capteur de pression (106B) est disposé sur la première électrode (105A) et/ou la seconde électrode (105B), et/ou le capteur capacitif (106D) est disposé sur la première électrode (105A) et/ou la seconde électrode (105B) ;
le capteur de pression (106B) et/ou le capteur capacitif (106D) sont/est configuré(s) pour détecter un état de contact de l'utilisateur avec la première électrode (105A) et/ou la seconde électrode (105B) ; et
le processeur (103) est spécifiquement configuré pour déterminer (S1402) la largeur de bande de fréquences sur la base de l'état de contact.

10. Dispositif portable (100) selon l'une quelconque des revendications 7 à 9, dans lequel le dispositif portable (100) comprend en outre un dispositif d'affichage ; et
avant que la première électrode (105A) détecte (S1401) le premier signal électrique, et que la seconde électrode (105B) détecte (S1401) le second signal électrique, le dispositif d'affichage affiche un écran de verrouillage à l'état éteint, un écran de verrouillage à l'état allumé, ou un écran d'accueil.

11. Dispositif portable (100) selon l'une quelconque des revendications 7 à 10, dans lequel le dispositif portable (100) comprend en outre un dispositif d'entrée (101), et le dispositif d'entrée (101) est configuré pour détecter une opération d'entrée ; et
le processeur (103) est en outre configuré pour activer une fonction de détection ECG automatique en réponse à l'opération d'entrée, de sorte que la première électrode (105A) et la seconde électrode (105B) sont toujours dans un état activé.

12. Dispositif portable (100) selon l'une quelconque des revendications 7 à 11, dans lequel le dispositif portable (100) comprend le dispositif d'affichage, et le dispositif d'affichage est configuré pour afficher une forme d'onde correspondant à l'ECG et des informations d'état de santé correspondant à l'ECG.

13. Produit-programme, dans lequel le produit-programme comprend des instructions, et lorsque les instructions sont exécutées sur un ordinateur, l'ordinateur peut mettre en œuvre le procédé selon l'une quelconque des revendications 1 à 6, dans lequel les instructions d'ordinateur sont exécutées sur le dispositif portable spécifique selon l'une ou plusieurs des revendications 7 à 12.
